# EUROPEAN PATENT SPECIFICATION

(11) **EP 2 063 710 B1**
(45) Date of publication and mention of the grant of the patent: **26.01.2011**
(21) Application number: 07837578.9
(22) Date of filing: 31.08.2007
(51) Int. Cl.: A01N 43/40, A61K 31/435, C07D 211/34

(54) **DIFLUORINATED PIPERIDINES FOR TREATMENT OF ALZHEIMER'S DISEASE AND RELATED CONDITIONS**
DIFLUORIERTE PIPERIDINE ZUR BEHANDLUNG VON MORBUS ALZHEIMER UND VERWANDTEN LEIDEN
PIPÉRIDINES DIFLUORÉES POUR LE TRAITEMENT DE LA MALADIE D'ALZHEIMER ET DES TROUBLES APPARENTÉS

(30) Priority: 07.09.2006 US 842727 P
(43) Date of publication of application: 03.06.2009
(73) Proprietor: Merck Sharp & Dohme Corp., Rahway, NJ 07065 (US)
(72) Inventor: STANTON, Matthew, G., Rahway, NJ 07065-0907 (US); MUNOZ, Benito, Newtonville, Massachusetts 02460 (US); SLOMAN, David, L., Rahway, NJ 07065-0907 (US); HUBBS, Jed, Rahway, NJ 07065-0907 (US); HAMBLETT, Christopher, Boston, Massachusetts 02132 (US)
(74) Representative: Buchan, Gavin MacNicol
(86) International application number: PCT/US2007/019127
(87) International publication number: WO 2008/030391

(56) References cited:
- WO-A-2006/043064
- WO-A1-2006/043064

## Description

This invention relates to compounds for use in therapeutic treatment of the human body. In particular, it provides difluorinated carboxy-functional 1,2-disubstituted piperidines and related compounds useful for treating diseases associated with the deposition of β-amyloid peptide in the brain, such as Alzheimer's disease, or of preventing or delaying the onset of dementia associated with such diseases.

Alzheimer's disease (AD) is the most prevalent form of dementia. Its diagnosis is described in the Diagnostic and Statistical Manual of Mental Disorders, 4th ed., published by the American Psychiatric Association (DSM-IV). It is a neurodegenerative disorder, clinically characterized by progressive loss of memory and general cognitive function, and pathologically characterized by the deposition of extracellular proteinaceous plaques in the cortical and associative brain regions of sufferers. These plaques mainly comprise fibrillar aggregates of β-amyloid peptide (Aβ). Aβ is formed from amyloid precursor protein (APP) via separate intracellular proteolytic events involving the enzymes β-secretase and γ-secretase. Variability in the site of the proteolysis mediated by γ-secretase results in Aβ of varying chain length, e.g. Aβ(1-38), Aβ(1-40) and Aβ(1-42). N-terminal truncations such as Aβ(4-42) are also found in the brain, possibly as a result of variability in the site of proteolysis mediated by β-secretase. For the sake of convenience, expressions such as "Aβ(1-40)" and "Aβ(1-42)" as used herein are inclusive of such N-terminal truncated variants. After secretion into the extracellular medium, Aβ forms initially-soluble aggregates which are widely believed to be the key neurotoxic agents in AD (see Gong et al, PNAS, 100 (2003), 10417-22), and which ultimately result in the insoluble deposits and dense neuritic plaques which are the pathological characteristics of AD.

Other dementing conditions associated with deposition of Aβ in the brain include cerebral amyloid angiopathy, hereditary cerebral haemorrhage with amyloidosis, Dutch-type (HCHWA-D), multi-infarct dementia, dementia pugilistica and Down syndrome.

Various interventions in the plaque-forming process have been proposed as therapeutic treatments for AD (see, for example, Hardy and Selkoe, Science, 297 (2002), 353-6). One such method of treatment that has been proposed is that of blocking or attenuating the production of Aβ for example by inhibition of β- or γ-secretase. It has also been reported that inhibition of glycogen synthase kinase-3 (GSK-3), in particular inhibition of GSK-3α, can block the production of Aβ (see Phiel et al, Nature, 423 (2003), 435-9). Other proposed methods of treatment include administering a compound which blocks the aggregation of Aβ, and administering an antibody which selectively binds to Aβ.

However, recent reports (Pearson and Peers, J. Physiol., 575.1 (2006), 5-10) suggest that Aβ may exert important physiological effects independent of its role in AD, implying that blocking its production may lead to undesirable side effects. Furthermore, γ-secretase is known to act on several different substrates apart from APP (e.g. notch), and so inhibition thereof may also lead to unwanted side effects. There is therefore an interest in methods of treating AD that do not suppress completely the production of Aβ, and do not inhibit the action of γ-secretase.

One such proposed treatment involves modulation of the action of γ-secretase so as to selectively attenuate the production of Aβ(1-42). This results in preferential secretion of the shorter chain isoforms of Aβ, which are believed to have a reduced propensity for self-aggregation and plaque formation, and hence are more easily cleared from the brain, and/or are less neurotoxic. Compounds showing this effect include certain non-steroidal antiinflammatory drugs (NSAIDs) and their analogues (see WO 01/78721 and US 2002/0128319 and Weggen et al Nature, 414 (2001) 212-16; Morihara et al, J. Neurochem., 83 (2002), 1009-12; and Takahashi et al, J. Biol. Chem., 278 (2003), 18644-70). Compounds which modulate the activity of PPARα and/or PPARδ are also reported to have the effect of lowering Aβ(1-42) (WO 02/100836). NSAID derivatives capable of releasing nitric oxide have been reported to show improved anti-neuroinflammatory effects and/or to reduce intracerebral Aβ deposition in animal models (WO 02/092072; Jantzen et al, J. Neuroscience, 22 (2002), 226-54). US 2002/0015941 teaches that agents which potentiate capacitative calcium entry activity can lower Aβ(1-42).

Further classes of compounds capable of selectively attenuating Aβ(1-42) production are disclosed on WO 2005/054193, WO 2005/013985, WO 2006/008558, WO 2005/108362 and WO 2006/043064. The aforementioned WO 2006/043064 discloses *inter alia* various N-substituted piperidinylacetic acid derivatives, but neither discloses nor suggests the compounds of the present invention.

The compounds of the present invention selectively attenuate Aβ(1-42) production with a reduced propensity for undesirable side effects.

According to the present invention there is provided a compound of formula I: 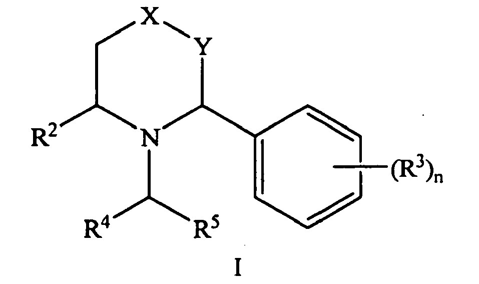 or a pharmaceutically acceptable salt or hydrate thereof; wherein:
n is 0, 1, 2 or 3;
one of X and Y represents CF₂ and the other represents CH-C(R¹)₂-Z;
Z represents CO₂H or a tetrazole ring;
each R¹ independently represents H or a non-aromatic hydrocarbon group of up to 6 carbon atoms; or the two R¹ groups complete a C₃₋₆alicyclic group;
R² represents H or phenyl which optionally bears up to 3 substituents independently selected from halogen, C₁₋₆alkyl bearing 0-3 fluorine substituents, C₁₋₆alkoxy bearing 0-3 fluorine substituents, and C₂₋₆alkenyl;
each R³ independently represents halogen, C₁₋₆alkyl bearing 0-3 fluorine substituents, C₁₋₆alkoxy bearing 0-3 fluorine substituents, or C₂₋₆alkenyl; and
R⁴ and R⁵ independently represent H or hydrocarbon of up to 12 carbon atoms which optionally bears up to 3 substituents selected from halogen, perfluoroC₁₋₄alkyl, CN, Si(C₁₋₄alkyl)₃, OH, C₁₋₄alkoxy and OCF₃.

Where a variable occurs more than once in formula I, the identity taken by said variable at any particular occurrence is independent of the identity taken at any other occurrence.

As used herein, the expression "hydrocarbon group" refers to groups consisting solely of carbon and hydrogen atoms. Unless indicated otherwise, such groups may comprise linear, branched or cyclic structures, singly or in any combination consistent with the indicated maximum number of carbon atoms, and may be saturated or unsaturated, including aromatic unless indicated otherwise.

As used herein, the expression "C₁₋ₓalkyl" where x is an integer greater than 1 refers to straight-chained and branched alkyl groups wherein the number of constituent carbon atoms is in the range 1 to x. Particular alkyl groups are methyl, ethyl, n-propyl, isopropyl and t-butyl. Derived expressions such as "C₂₋₆alkenyl", "hydroxyC₁₋₆alkyl", "heteroarylC₁₋₆alkyl", "C₂₋₆alkynyl" and "C₁₋₆alkoxy" are to be construed in an analogous manner.

The expression "perfluoroC₁₋₄alkyl" refers to linear or branched alkyl groups of up to 4 carbon atoms in which all the hydrogen atoms are replaced by fluorine atoms.

The expression "C₃₋₆alicyclic" refers to cyclic non-aromatic hydrocarbon groups containing from 3 to 6 ring carbon atoms. Examples include cyclopropyl, cyclobutyl, cyclopentenyl, cyclopentyl and cyclohexyl.

The term "halogen" as used herein includes fluorine, chlorine, bromine and iodine, of which fluorine and chlorine are preferred unless otherwise indicated.

For use in medicine, the compounds of formula I may be in the form of pharmaceutically acceptable salts. Other salts may, however, be useful in the preparation of the compounds of formula I or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, benzenesulphonic acid, fumaric acid, maleic acid, succinic acid, acetic acid, benzoic acid, oxalic acid, citric acid, tartaric acid, carbonic acid or phosphoric acid. Alternatively, a pharmaceutically acceptable salt may be formed by neutralisation of a carboxylic acid group with a suitable base. Examples of pharmaceutically acceptable salts thus formed include alkali metal salts such as sodium or potassium salts; ammonium salts; alkaline earth metal salts such as calcium or magnesium salts; and salts formed with suitable organic bases, such as amine salts (including pyridinium salts) and quaternary ammonium salts.

It is to be understood that all the stereoisomeric forms encompassed by formula I, both optical and geometrical, fall within the scope of the invention, singly or as mixtures in any proportion. Thus the moieties: 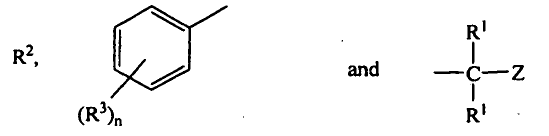 may be in *cis-* or trans-configurations with respect to the piperidine ring. Furthermore, a given compound in a given *cis-* or trans-configuration will have two enantiomeric forms, both of which are within the scope of the invention, whether as single homochiral compounds or as racemic mixtures in any proportion. For the avoidance of any doubt, structural formulae such as (A): 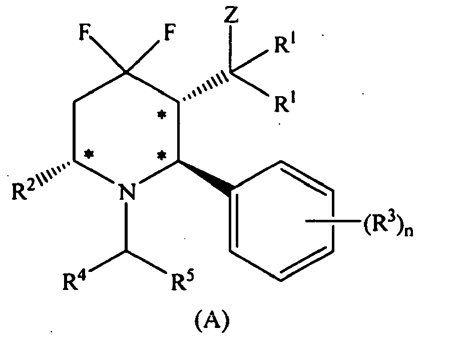 as used herein shall be taken to be definitive of the relative configurations of the carbon atoms marked with asterisks, but not their absolute configurations, unless expressly stated otherwise.

In formula I, one of X and Y represents CF₂ and the other represents CH-C(R¹)₂-Z. Thus, in one embodiment of the invention X is CF₂, Y is CH-C(R¹)₂-Z and the compounds are therefore 4,4-difluoropiperidine derivatives. In an alternative embodiment, Y is CF₂, X is CH-C(R¹)₂-Z and the compounds are therefore 3,3-difluoropiperidine derivatives.

Z represents CO₂H or a tetrazole ring, in particular, Z represents CO₂H or 1,2,3,4-tetrazol-5-yl, but preferably represents CO₂H.

Each R¹ independently represents H or a non-aromatic hydrocarbon group of up to 6 carbon atoms; or the two R¹ groups complete a C₃₋₆alicyclic group (such as cyclopropyl, cyclobutyl, cyclopentenyl or cyclopentyl). In one embodiment, one R¹ group is H and the other is H or C₁₋₆alkyl such as methyl, ethyl, propyl or butyl. In another embodiment, both R¹ groups represent methyl or together complete an alicyclic group. In a further embodiment, both R¹ groups represent H.

R² represents H or phenyl which is optionally substituted as detailed previously. Typically, R² represents H or mono- or disubsituted phenyl. Examples of substituents include 2-CF₃, 3-CF₃, 4-CF₃, 2,4-di(CF₃), 2-F-4-CF₃, 4-OCF₃, 4-allyl, 4-n-propyl, 4-isopropropyl and 4-tert-butyl. In a particular embodiment R² represents H or 4-trifluoromethylpenyl.

In formula I, n is preferably 1 or 2, most preferably 1. Each R³ independently represents halogen (especially F), C₁₋₆alkyl bearing 0-3 fluorine substituents, C₁₋₆alkoxy bearing 0-3 fluorine substituents, or C₂₋₆alkenyl. When one R³ is present, it is very suitably (but not necessarily) attached in the 4-position. Typical identities for (R³)ₙ include 2-CF₃, 3-CF₃, 4-CF₃, 2,4-di(CF₃), 2-F-4-CF₃, 4-OCF₃, 4-allyl, 4-n-propyl, 4-isopropyl and 4-tert-butyl. In one embodiment, (R³)ₙ represents 4-CF₃ or 4-n-propyl, in particular 4-CF₃.

R⁴ and R⁵ independently represent H or a hydrocarbon group of up to 12 carbon atoms which optionally bears up to 3 substituents selected from halogen, perfluoroC₁₋₄alkyl, CN, Si(C₁₋₄alkyl)₃, OH, C₁₋₄alkoxy and OCF₃. Preferably R⁴ and R⁵ do not both represent H, and in a particular embodiment neither R⁴ nor R⁵ represents H. In a further embodiment, if R⁴ and/or R⁵ represents H, R² represents optionally-substituted phenyl.

Hydrocarbon groups represented by R⁴ and/or R⁵ may be linear, branched or cyclic, or may comprise any combination of linear, branched and cyclic moieties having a maximum of 12 carbon atoms. Said hydrocarbon groups may be fully saturated or may contain one or more double or triple bonds, or any combination thereof, including aromatic rings. However, in a particular embodiment, not more than one of R⁴ and R⁵ comprises an aromatic ring. Typical examples of hydrocarbon groups represented by R⁴ and/or R⁵ include linear or branched C₁₋₁₂alkyl, alkenyl and alkynyl groups, C₃₋₆alicyclic groups, C₃₋₆alicyclicC₁₋₄alkyl groups, phenyl groups, phenyl C₁₋₄alkyl groups, phenylC₂₋₄alkynyl groups and C₃₋₆alicyclicC₂₋₄alkynyl groups.

Said hydrocarbon groups may be unsubstituted or may bear up to 3 substituents as defined previously. However, when more than one substituent is present, said substituents are typically attached to a phenyl ring. In a particular embodiment, hydrocarbon groups represented by R⁴ or R⁵ bear not more than one substituent. Preferred substituents (if present) include halogen (e.g. Cl or F), C₁₋₄perfluoroalkyl (eg CF₃ or C₂F₅) and Si(C₁₋₄alkyl)₃ (e.g. trimethylsilyl).

Examples of groups represented by R⁴ and/or R⁵ include H, methyl, ethyl, n-propyl, isopropyl, n-butyl, isobutyl, t-butyl, 3-methylbutyl, 2,2-dimethylpropyl, 2-ethylbutyl, 4-methylpentyl, 3,3-dimethylbutyl, 4,4-dimethylpentyl, 3-methyl-1-butenyl, 3-methyl-3-butenyl, 3-methyl-3-butene-1-ynyl, 4-methyl-1-pentynyl, 3,3-dimethyl-1-butynyl, 2-(trimethylsilyl)ethyl, 2,2,2-trifluoroethyl, 3,3,3-trifluoropropyl, 4,4,4-trifluorobutyl, 2-phenylethyl, 3-methoxyprop-1-ynyl, cyclohexylethynyl, cyclopropylethynyl, 1-methyl-3,3,3-trifluoropropyl, 2,2,3,3,3-pentafluoropropyl, hydroxymethyl, isopropoxymethyl, difluoromethoxymethyl, 4-hydroxy-3-methyl-1-butynyl, 4-hydroxy-3-methylbutyl, 2-cyclopropylethyl, 2-cyclohexylethyl, 2-(cyclohexen-1-yl)ethyl, 2-(1-hydroxycyclopentyl)ethyl, 2-(1-hydroxycyclohexyl)ethyl, 4-(trifluoromethyl)phenyl, 4-(trifluoromethyl)phenylethynyl, 2-(3-fluorophenyl)ethyl, 2-(3,5-difluorophenyl)ethyl, 2-(2,4-difluorophenyl)ethyl, 2-(3-methylphenyl)ethyl, 2-(4-t-butylphenyl)ethyl, 2-[3-(trifluoromethyl)phenyl]ethyl and 2-[4-(trifluoromethyl)phenyl]ethyl.

Preferred examples of groups represented by R⁴ and/or R⁵ include H, 3-methylbutyl, 2,2-dimethylpropyl, 3,3-dimethylbutyl, 4,4-dimethylpentyl, 3-methyl-3-butenyl, 3-methyl-3-butene-1-ynyl, 3,3-dimethyl-1-butynyl, 2-(trimethylsilyl)ethyl, 3,3,3-trifluoropropyl, 4-(trifluoromethyl)phenyl, 4-(trifluoromethyl)phenylethynyl, and 2-[4-(trifluoromethyl)phenyl]ethyl.

A first subset of the compounds according to the invention consists of the compounds of formula II: 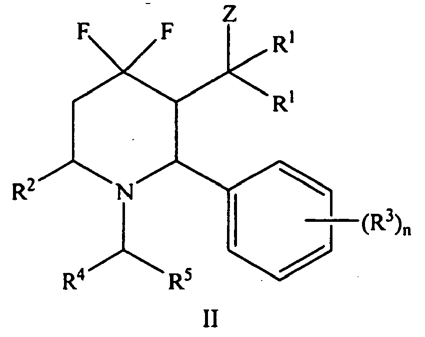 and the pharmaceutically acceptable salts and hydrates thereof;
wherein n, Z, R¹, R², R³, R⁴ and R⁵ have the same definitions and preferred identities as before.

In a particular embodiment of this subset, the relative stereochemical configurations of the substituents on the piperidine ring are as shown in formula IIA:

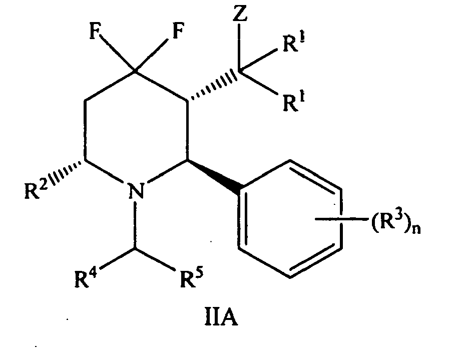

Specific examples of compounds in accordance with formula II include those in which Z is CO₂H, each R¹ is H, (R³)ₙ is 4-CF₃, and R², R⁴ and R⁵ are as shown in the following table:

| **R²** | **R⁴** | **R⁵** |
|---|---|---|
| H | CH₂CH₂CF₃ | C≡C-C(Me)=CH₂ |
| H | 4-CF₃-C₆H₅ | C≡C-C(Me)₃ |
| H | 4-CF₃-C₆H₅ | CH₂CH₂-C(Me)₃ |
| H | CH₂CH₂CF₃ | C≡C-C(Me)₃ |
| H | CH₂CH₂CF₃ | CH₂CH₂-C(Me)₃ |
| H | CH₂CH₂CF₃ | CH=CH-C(Me)₃ |
| H | CH₂CH₂-C(Me)=CH₂ | C≡C-C(Me)=CH₂ |
| H | 4-CF₃-C₆H₅ | CH₂CH₂CH(Me)₂ |
| H | CH₂CH₂CH(Me)₂ | CH₂CH₂CH(Me)₂ |
| H | CH₂-C(Me)₃ | C≡C-C(Me)₃ |
| H | CH₂-C(Me)₃ | CH₂CH₂-C(Me)₃ |
| H | CH₂-C(Me)₃ | CH=CH-C(Me)₃ |
| H | CH₂-C(Me)₃ | C≡C-C₆H₄-4-CF₃ |
| H | CH₂-C(Me)₃ | CH₂CH₂-C₆H₄-4-CF₃ |
| H | CH₂CH₂-C(Me)₃ | CH₂CH₂-C(Me)₃ |
| H | CH₂CH₂-C(Me)₃ | CH₂CH₂CH₂-C(Me)₃ |
| H | CH₂CH₂CH₂-C(Me)₃ | C≡C-C(Me)₃ |
| H | CH₂CH₂Si(Me)₃ | C≡C-C(Me)₃ |
| H | CH₂CH₂CF₃ | CH₂CH₂Si(Me)₃ |
| 4-CF₃-C₆H₅ | H | C≡C-C(Me)₃ |
| 4-CF₃-C₆H₅ | H | CH₂CH₂-C(Me)₃ |
| H | CH₂CH₂CH₂CF₃ | C≡C-cyclopropyl |

A second subset of the compounds according to the invention consists of the compounds of formula III: 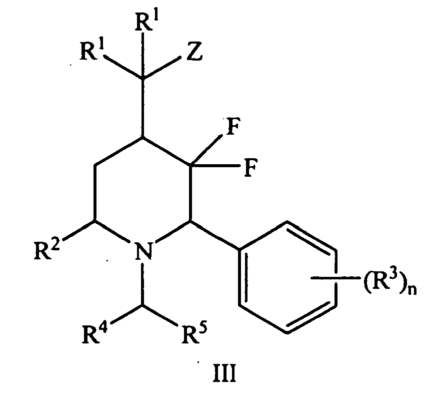 and the pharmaceutically acceptable salts and hydrates thereof;
wherein n, Z, R¹, R², R³, R⁴ and R⁵ have the same definitions and preferred identities as before.

In a particular embodiment of this subset, the relative stereochemical configurations of the substituents on the piperidine ring are as shown in formula IIIA:

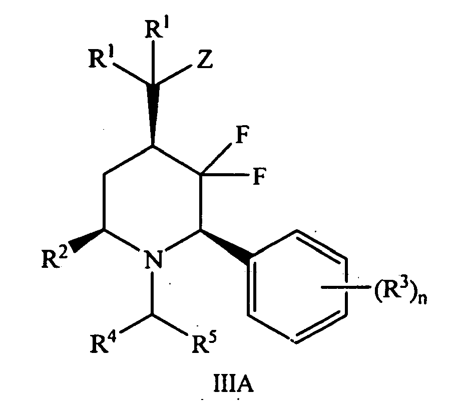

Specific examples of compounds in accordance with formula II include those in which Z is CO₂H, each R¹ is H, (R³)ₙ is 4-CF₃, and R², R⁴ and R⁵ are as shown in the following table:

| **R²** | **R⁴** | **R⁵** |
|---|---|---|
| H | CH₂CH₂CF₃ | CH₂CH₂-C(Me)₃ |
| H | 4-CF₃-C₆H₅ | CH₂CH₂-CH(Me)₂ |
| H | CH₂CH₂CF₃ | CH₂CH₂-SiMe₃ |

The compounds of formula I in which Z is CO₂H are typically obtained by hydrolysis of the corresponding esters (1): 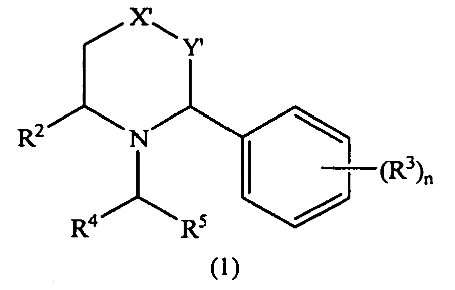 where one of X' and Y' represents CF₂ and the other represents C(R¹)₂-CO₂R where R represents C₁₋₆alkyl such as methyl or ethyl, and n, R¹, R², R³, R⁴ and R⁵ have the same meanings as before. The hydrolysis is typically carried out by refluxing with LiOH in aqueous THF or with NaOH or KOH in methanol.

Corresponding compounds in which Z represents 1*H*-tetrazol-5-yl are obtainable by conversion of the esters (1) to the corresponding nitriles, followed by treatment with azidotrimethylsilane in refluxing toluene in the presence of tributyltin oxide. The conversion to the nitrile may be carried out by adding trimethylaluminium to a suspension of ammonium chloride in toluene, then adding the ester (1), refluxing the mixture, and treating with solid potassium sodium tartrate.

Esters (1) in which X' represents CF₂ may be obtained by reaction of compounds (2) with R⁴R⁵CH-L: 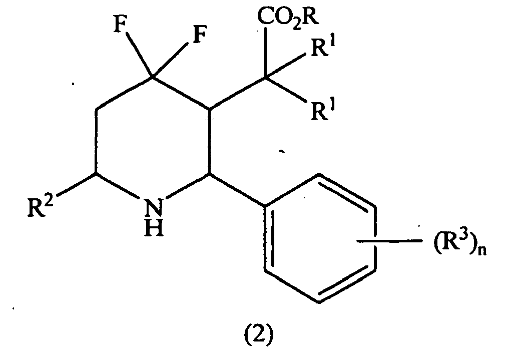 where L is a leaving group such as halide (especially bromide or iodide), tosylate, mesylate or triflate, and R, n, R¹, R², R³, R⁴ and R⁵ have the same meanings as before. Normal alkylating conditions may be employed, e.g. heating in DMF solution in the presence of base such as potassium carbonate.

Alternatively, compounds (2) may undergo reductive alkylation with precursors of the group R⁴R⁵CH- which contain an aldehyde or ketone functionality. In such cases, the compound (2) may be refluxed with R⁴-CO-R⁵ in toluene in the presence of an acid catalyst, with azeotropic removal of water, and the resulting adduct reduced using sodium triacetoxyborohydride. In a preferred variant of this route, useful when R⁴ is other than H and R⁵ is an alkyn-1-yl group, a compound (2) is reacted with R⁴-CHO and R⁵-H in the presence of gold(III) bromide, e.g. via microwave heating at 70°C in water.

In another variant, the compound (2), R⁴-CHO and benzotriazole are refluxed in toluene with azeotropic removal of water, and the resulting adduct reacted with R⁵-Zn-Hal where Hal represents halide (preferably chloride). The reaction is suitably carried out in an anhydrous aprotic solvent such as dichloromethane at reduced temperature, e.g. below 10°C.

Compounds (2) are obtainable by fluorination of piperidones (3), followed by removal of the Cbz protecting group: 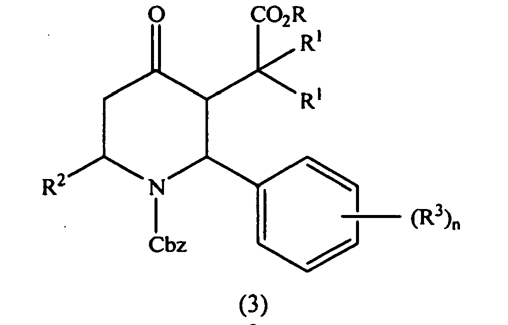 where Cbz represents benzyloxycarbonyl and n, R, R¹, R² and R³ are as defined previously. The fluorination may be carried out in dichloromethane at 0°C to ambient temperature using [bis(2-methoxyethyl)amino]sulfur trifluoride (Deoxofluor™), and the Cbz group is removable by hydrogenation over Pd(OH)₂ in methanol.

Compounds (3) may be obtained from the unsaturated derivatives (4) by borohydride reduction (when R² is H) or by treatment with the appropriate arylcuprate (when R² is optionally-substituted phenyl): 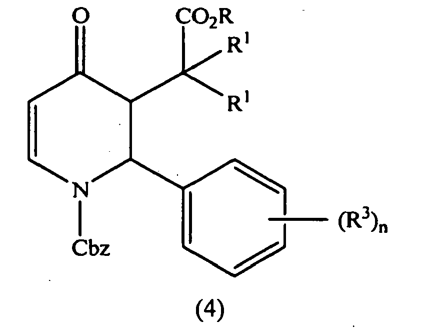 where Cbz, n, R, R¹, R² and R³ are as defined previously. The borohydride reduction may be carried out in THF at -78°C using L-Selectride™, and the reaction with arylcuprate may be carried out in THF at 0°C, the arylcuprate being generated *in situ* from R²MgBr and CuI.

Compounds (4) are obtainable by alkylation of compounds (5) with L-C(R¹)₂-CO₂R; 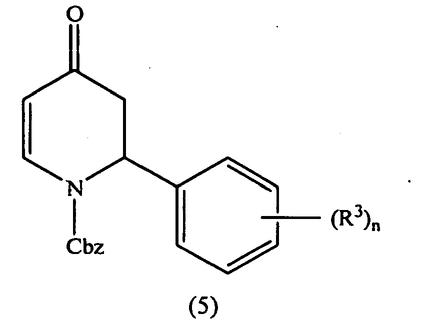 where Cbz, L, n, R¹, R² and R³ have the same meanings as before. Preferably, L represents Br or I. The alkylation may be carried out in THF at -78 to 0°C in the presence of strong base such as lithium bis(dimethylsilyl)amide. This step is typically carried out using methyl bromoacetate as the alkylating agent (i.e. both R¹ groups represent H). Compounds (4) in which one or both R¹ groups is not H may be obtained via separate mono-or dialkylation of the corresponding compounds in which both R¹ groups are H.

Compounds (5) are obtainable by reaction of 4-methoxypyridine with benzyl chloroformate and (R³)ₙC₆H₍₅₋ₙ₎MgBr by the procedure described in Commins, J. Heterocyclic Chem., (1999), 36, 1491-1500 (see also WO 2006/043064).

Esters of formula (1) in which Y' represents CF₂ and both R¹ groups are H are obtainable by hydrogenation of compounds (6): 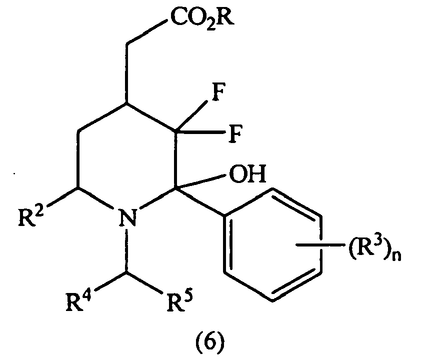 where R, n, R², R³, R⁴ and R⁵have the same meanings as before. The hydrogenation may be carried out using borane-THF complex in THF at -78°C to ambient temperature.

Esters of formula (1) in which one or both R¹ groups is other than H may be obtained by alkylation of esters (1) in which both R¹ groups are H.

Compounds (6) are obtainable via reaction of compounds (7) with 1-chloromethyl-4-fluoro-1,4-diazoniabicyclo[2.2.2]octane bis(tetrafluoroborate) (Selectfluor™) : 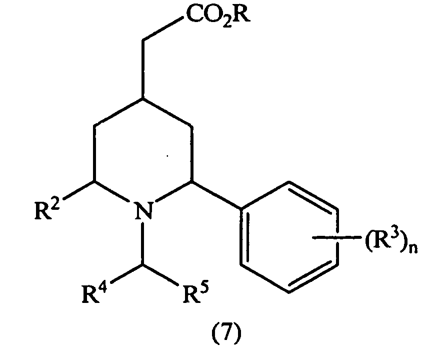 where R, n, R², R³, R⁴ and R⁵ have the same meanings as before. The reaction is typically carried out in DMF followed by quenching in water:

Compounds (7) are obtainable by N-alkylation of piperidines (8): 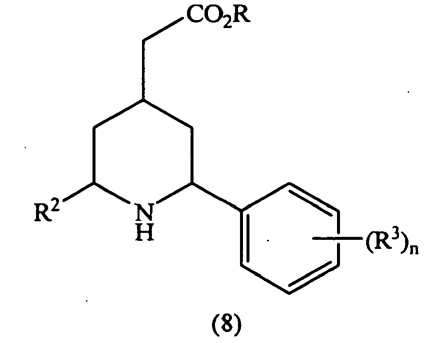 where R, n, R² and R³ have the same meanings as before, using any of the procedures described above for the conversion of compounds (2) to compounds (1).

The synthesis of piperidines (8) in which R² is H is described in WO 2006/043064 The corresponding compounds in which R² is other than H may be obtained analogously.

A given compound in accordance with formula I, or a precursor thereof, may be converted to a different compound in accordance with formula I, or precursor thereof, by means of the standard techniques of bond formation or cleavage known to those skilled in the art of organic synthesis. For example, esters of formula (1) in which at least one R¹ is other than H may be prepared by alkylation of the corresponding compounds in which each R¹ is H by standard methods. Similarly, esters (1) in which R⁴ and/or R⁵ comprise unsaturation may be hydrogenated (e.g. over Raney Ni) to provide partially or fully saturated analogs.

Where they are not themselves commercially available, the starting materials for the synthetic schemes described above are available by straightforward chemical modifications of commercially available materials.

Certain compounds according to the invention may exist as optical isomers due to the presence of one or more chiral centres or because of the overall asymmetry of the molecule. Such compounds may be prepared in racemic form, or individual enantiomers may be prepared either by enantiospecific synthesis or by resolution. The novel compounds may, for example, be resolved into their component enantiomers by standard techniques such as preparative HPLC, or the formation of diastereomeric pairs by salt formation with an optically active acid, such as di-p-toluoyl-D-tartaric acid and/or di-p-toluoyl-L-tartaric acid, followed by fractional crystallisation and regeneration of the free base. The novel compounds may also be resolved by formation of diastereomeric esters or amides, followed by chromatographic separation and removal of the chiral auxiliary. Alternatively, racemic intermediates in the preparation of compounds of formula I may be resolved by the aforementioned techniques, and the desired enantiomer used in subsequent steps. For example, racemic piperidine derivatives (2) may be resolved by chiral chromatography, and racemic piperidine derivatives (8) may be resolved via salt formation with L-mandelic acid.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 3rd ed., 1999. The protecting groups may be removed at a convenient subsequent stage using methods known from the art.

The compounds of the invention have the useful property of modifying the action of γ-secretase on amyloid precursor protein so as to selectively reduce the formation of the 1-42 isoform of Aβ, and hence find use in the development of treatments for diseases mediated by Aβ(1-42), in particular diseases involving deposition of β-amyloid in the brain.

According to a further aspect of the invention there is provided the use of a compound according to formula I as defined above, or a pharmaceutically acceptable salt or hydrate thereof, for the manufacture of a medicament for treatment or prevention of a disease associated with the deposition of β-amyloid in the brain.

The disease associated with deposition of Aβ in the brain is typically Alzheimer's disease (AD), cerebral amyloid angiopathy, HCHWA-D, multi-infarct dementia, dementia pugilistica or Down syndrome, preferable AD.

In a further aspect, the invention provides the use of a compound of Formula I as defined above, or a pharmaceutically acceptable salt or hydrate thereof, in the manufacture of a medicament for treating, preventing or delaying the onset of dementia associated with Alzheimer's disease, cerebral amyloid angiopathy, HCHWA-D, muiti-infaret dementia, dementia pugilistica or Down syndrome.

The compounds of Formula 1 modulate the action of γ-secretase so as to selectively attenuate production of the (1-42) isoform of Aβ without significantly low-ering production of the shorter chain isoforms such as Aβ(1-40). This results in secretion of Aβ which has less tendency to seif-aggrsgate and form insoluble deposits, is more easily cleared from the brain, and/or is less neurotoxic. Therefore, a further aspect of the invention provides a method for retarding, arresting or preventing the accumulation of Aβ in the brain comprising administering to a subject in need thereof a therapeutically effective amount of a compound of Formula I as defined above or a pharmaceutically acceptable salt thereof.

Because the compounds of formula I modulate the activity of γ-secretase, as opposed to suppressing said activity, it is believed that the therapeutic benefits described above will be obtained with a reduced risk of side effects, e.g. those that might arise from a disruption of other signaling pathways (e.g. Notch) which are controlled by γ-secretase.

In one embodiment of the invention, the compound of Formula I is administered to a patient suffering from AD, cerebral amyloid angiopathy, HCHWA-D, multi-infarct dementia, dementia pugilistica or Down syndrome, preferably AD.

In an alternative embodiment of the invention, the compound of Formula I is administered to a patient suffering from mild cognitive impairment or age-related cognitive decline. A favourable outcome of such treatment is prevention or delay of the onset of AD. Age-related cognitive decline and mild cognitive impairment (MCI) are conditions in which a memory deficit is present, but other diagnostic criteria for dementia are absent (Santacruz and Swagerty, American Family Physician, 63 (2001), 703-13). (See also "The ICD-10 Classification of Mental and Behavioural Disorders", Geneva: World Health Organisation, 1992, 64-5). As used herein, "age-related cognitive decline" implies a decline of at least six months' duration in at least one of: memory and learning; attention and concentration; thinking; language; and visuospatial functioning and a score of more than one standard deviation below the norm on standardized neuropsychologic testing such as the MMSE. In particular, there may be a progressive decline in memory. In the more severe condition MCI, the degree of memory impairment is outside the range considered normal for the age of the patient but AD is not present. The differential diagnosis of MCI and mild AD is described by Petersen et al., Arch. Neurol., 56 (1999), 303-8. Further information on the differential diagnosis of MCI is provided by Knopman et al, Mayo Clinic Proceedings, 78 (2003), 1290-1308. In a study of elderly subjects, Tuokko et al (Arch, Neurol., 60 (2003) 577-82) found that those exhibiting MCI at the outset had a three-fold increased risk of developing dementia within 5 years.

Grundman et al (J Mol. Neurosci., 19 (2002), 23-28) report that lower baseline hippocampal volume in MCI patients is a prognostic indicator for subsequent AD. Similarly, Andreasen et al (Acta Neurol. Scand, 107 (2003) 47-51) report that high CSF levels of total tau, high CSF levels of phospho-tau and lowered CSF levels of Aβ42 are all associated with increased risk of progression from MCI to AD.

Within this embodiment, the compound of Formula I is advantageously administered to patients who suffer impaired memory function but do not exhibit symptoms of dementia. Such impairment of memory function typically is not attributable to systemic or cerebral disease, such as stroke or metabolic disorders caused by pituitary dysfunction. Such patients may be in particular people aged 55 or over, especially people aged 60 or over, and preferably people aged 65 or over. Such patients may have normal patterns and levels of growth hormone secretion for their age. However, such patients may possess one or more additional risk factors for developing Alzheimer's disease. Such factors include a family history of the disease; a genetic predisposition to the disease; elevated serum cholesterol; and adult-onset diabetes mellitus.

In a particular embodiment of the invention, the compound of Formula I is administered to a patient suffering from age-related cognitive decline or MCI who additionally possesses one or more risk factors for developing AD selected from: a family history of the disease; a genetic predisposition to the disease; elevated serum cholesterol; adult-onset diabetes mellitus; elevated baseline hippocampal volume; elevated CSF levels of total tau; elevated CSF levels of phospho-tau; and lowered CSF levels of Aβ(1-42),

A genetic predisposition (especially towards early onset AD) can arise from point mutations in one or more of a number of genes, including the APP, presenilin-1 and presenilin-2 genes. Also, subjects who are homozygous for the ε4 isoform of the apolipoprotein E gene are at greater risk of developing AD.

The patient's degree of cognitive decline or impairment is advantageously assessed at regular intervals before, during and/or after a course of treatment in accordance with the invention, so that changes therein may be detected, e.g. the slowing or halting of cognitive decline. A variety of neuropsychological tests are known in the art for this purpose, such as the Mini-Mental State Examination (MMSE) with norms adjusted for age and education (Folstein et al., J. Psych. Res., 12 (1975), 196-198, Anthony et al., Psychological Med., 12 (1982), 397-408; Cockrell et al., Psychopharmacology, 24 (1988), 689-692; Crum et al., J. Am. Med. Assoc'n. 18 (1993), 2386-2391). The MMSE is a brief, quantitative measure of cognitive status in adults. It can be used to screen for cognitive decline or impairment, to estimate the severity of cognitive decline or impairment at a given point in time, to follow the course of cognitive changes in an individual over time, and to document an individual's response to treatment. Another suitable test is the Alzheimer Disease Assessment Scale (ADAS), in particular the cognitive element thereof (ADAS-cog) (See Rosen et al., Am. J Psychiatry, 141 (1984), 1356-64).

The compounds of Formula I are typically used in the form of pharmaceutical compositions comprising one or more compounds of Formula I and a pharmaceutically acceptable carrier. Accordingly, in a further aspect the invention provides a pharmaceutical composition comprising a compound of formula I as defined above, or a pharmaceutically acceptable salt thereof, and a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. The principal active ingredient typically is mixed with a pharmaceutical carrier, e.g. conventional tableting ingredients such as com starch, lactose, sucrose, sorbitol, talc, stearic acid, magnesium stearate and dicalcium phosphate, or gums, dispersing agents, suspending agents or surfactants such as sorbitan monooleate and polyethylene glycol, and other pharmaceutical diluents, e.g. water, to form a homogeneous preformulation composition containing a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This preformulation composition is then subdivided into unit dosage forms of the type described above containing from 0.1 to about 500 mg of the active ingredient of the present invention. Typical unit dosage forms contain from 1 to 100 mg, for example 1, 2, 5, 10, 25, 50 or 100 mg, of the active ingredient. Tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compositions useful in the present invention may be incorporated for administration orally or by injection include aqueous solutions, liquid- or gel-filled capsules, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, poly(ethylene glycol), poly(vinylpyrrolidone) or gelatin.

For treating or preventing Alzheimer's disease, a suitable dosage level is about 0.01 to 250 mg/kg per day, preferably about 0.01 to 100 mg/kg per day, and more preferably about 0.05 to 50 mg/kg of body weight per day, of the active compound. The compounds may be administered on a regimen of 1 to 4 times per day. In some cases, however, a dosage outside these limits may be used.

The compounds of Formula I optionally may be administered in combination with one or more additional compounds known to be useful in the treatment or prevention of AD or the symptoms thereof. Such additional compounds thus include cognition-enhancing drugs such as acetylcholinesterase inhibitors (e.g. donepezil and galanthamine), NMDA antagonists (e.g. memantine) or PDE4 inhibitors (e.g. Ariflo™ and the classes of compounds disclosed in WO 03/018579, WO 01/46151, WO 02/074726 and WO 02/098878). Such additional compounds also include cholesterol-lowering drugs such as the statins, e.g. simvastatin. Such additional compounds similarly include compounds known to modify the production or processing of Aβ in the brain ("amyloid modifiers"), such as compounds which inhibit the secretion of Aβ (including γ-secretase inhibitors, β-secretase inhibitors, and GSK-3α inhibitors), compounds which inhibit the aggregation of Aβ, and antibodies which selectively bind to Aβ. Such additional compounds also include growth hormone secretagogues, as disclosed in WO 2004/110443.

In this embodiment of the invention, the amyloid modifier may be a compound which inhibits the secretion of Aβ, for example an inhibitor of γ-secretase (such as those disclosed in WO 01/90084, WO 02/30912, WO 01/70677, WO 03/013506, WO 02/36555, WO 03/093252, WO 03/093264, WO 03/093251, WO 03/093253, WO 2004/039800, WO 2004/039370, WO 2005/030731, WO 2005/014553, WO 2004/089911, WO 02/081435, WO 02/081433, WO 03/018543, WO 2004/031137, WO 2004/031139, WO 2004/031138, WO 2004/101538, WO 2004/101539 and WO 02/47671), or a β-secretase inhibitor (such as those disclosed in WO 03/037325, WO 03/030886, WO 03/006013, WO 03/006021, WO 03/006423, WO 03/006453, WO 02/002122, WO 01/70672, WO 02/02505, WO 02/02506, WO 02/02512, WO 02/02520, WO 02/098849 and WO 02/100820), or any other compound which inhibits the formation or release of Aβ, including those disclosed in WO 98/28268, WO 02/47671, WO 99/67221, WO 01/34639, WO 01/34571, WO 00/07995, WO 00/38618, WO 01/92235, WO 01/77086, WO 01/74784, WO 01/74796, WO 01/74783, WO 01/60826, WO 01/19797, WO 01/27108, WO 01/27091, WO 00/50391, WO 02/057252, US 2002/0025955 and US2002/0022621, and also including GSK-3 inhibitors, particularly GSK-3α inhibitors, such as lithium, as disclosed in Phiel et al, Nature, 423 (2003), 435-9.

Within this embodiment, the amyloid modifier is advantageously a γ-secretase inhibitor, preferred examples of which include a compound of formula XI: 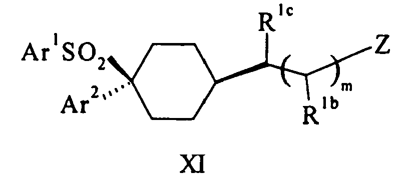 wherein m, Z, R^{1b}, R^{1c}, Ar¹ and Ar² are as defined in WO 03/018543;
or a pharmaceutically acceptable salt thereof.

Such compounds may be prepared as described in WO 03/018543. Preferred examples include those defined by formula XIa: 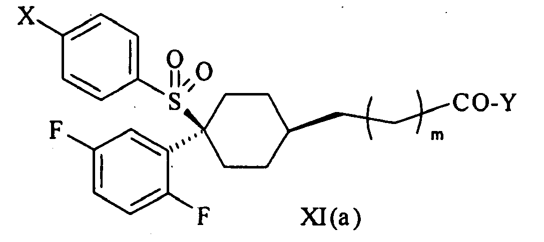 and the pharmaceutically acceptable salts thereof, wherein m is 0 or 1, X is Cl or CF₃, and Y is OH, OC₁₋₆alkyl, NH₂ or NHC₁₋₆alkyl. Particular examples include those in which m is 1 and Y is OH (or the sodium salts thereof), and those in which m is 0 and Y is NH₂ or NHC₁₋₆alkyl.

Another preferred class of γ-secretase inhibitors for use in this embodiment of the invention is that defined by formula XII: 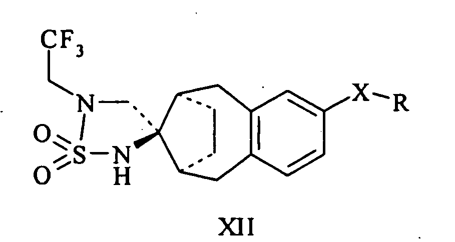 wherein X and R are as defined in WO 03/093252;
or a pharmaceutically acceptable salt thereof.

X is very aptly 5-substituted-thiazol-2-yl, 5-substituted-4-methylthiazol-2-yl, 5-substituted-1-methylpyrazol-3-yl, 1-substituted-imidazol-4-yl or 1-substituted-1,2,4-triazol-3-yl. Preferably, R represents optionally-substituted phenyl or heteroaryl such as phenyl, monohalophenyl, dihalophenyl, trihalophenyl, cyanophenyl, methylphenyl, methoxyphenyl, trifluoromethylphenyl, trifluoromethoxyphenyl, pyridyl, monohalopyridyl and trifluoromethylpyridyl, wherein "halo" refers to fluoro or chloro. Particularly preferred identities of R-X- include 5-(4-fluorophenyl)-1-methylpyrazol-3-yl, S-(4-chlorophenyl)-1-methylpyrazol-3-yl and 1-(4-fluorophenyl)imidazol-4-yl. Such compounds may be prepared by methods disclosed in WO 03/093252.

Alternatively, the amyloid modifier may be a compound which inhibits the aggregation of Aβ or otherwise attenuates is neurotoxicicity. Suitable examples include chelating agents such as clioquinol (Gouras and Beal, Neuron, 30 (2001), 641-2) and the compounds disclosed in WO 99/16741, in particular that known as DP-109 (Kalendarev et al, J. Pharm. Biomed. Anal., 24 (2001), 967-75). Other inhibitors of Aβ aggregation suitable for use in the invention include the compounds disclosed in WO 96/28471, WO 98/08868 and WO 00/052048, including the compound known as Apan™ (Praecis); WO 00/064420, WO 03/017994, WO 99/59571 (in particular 3-aminopropane-1-sulfonic acid, also known as tramiprosate or Alzhemed™); WO 00/149281 and the compositions known as PTI-777 and PTI-00703 (ProteoTech); WO 96/39834, WO 01/83425, WO 01/55093, WO 00/76988, WO 00/76987, WO 00/76969, WO 00/76489, WO 97/26919, WO 97/16194, and WO 97/16191. Further examples include phytic acid derivatives as disclosed in US 4,847,082 and inositol derivatives as taught in US 2004/0204387.

Alternatively, the amyloid modifier may be an antibody which binds selectively to Aβ. Said antibody may be polyclonal or monoclonal, but is preferably monoclonal, and is preferably human or humanized. Preferably, the antibody is capable of sequestering soluble Aβ from biological fluids, as described in WO 03/016466, WO 03/016467, WO 03/015691 and WO 01/62801. Suitable antibodies include humanized antibody 266 (described in WO 01/62801) and the modified version thereof described in WO 03/016466.

As used herein, the expression "in combination with" requires that therapeutically effective amounts of both the compound of Formula I and the additional compound are administered to the subject, but places no restriction on the manner in which this is achieved. Thus, the two species may be combined in a single dosage form for simultaneous administration to the subject, or may be provided in separate dosage forms for simultaneous or sequential administration to the subject. Sequential administration may be close in time or remote in time, e.g. one species administered in the morning and the other in the evening. The separate species may be administered at the same frequency or at different frequencies, e.g. one species once a day and the other two or more times a day. The separate species may be administered by the same route or by different routes, e.g. one species orally and the other parenterally, although oral administration of both species is preferred, where possible. When the additional compound is an antibody, it will typically be administered parenterally and separately from the compound of Formula I.

In a further aspect, the invention provides the combination of a compound of formula I or a pharmaceutically acceptable salt or hydrate thereof and a compound of formula XI(a) or a pharmaceutically acceptable salt thereof for use in treatment or prevention of a disease associated with deposition of β-amyloid in the brain. Said use may involve the simultaneous or separate administration of the respective compounds to a patient in need of such treatment or prevention.

In a further aspect, the invention provides a pharmaceutical composition comprising, in a pharmaceutically acceptable carrier, a compound of formula I or a pharmaceutically acceptable salt or hydrate thereof and a compound of formula XI(a) or a pharmaceutically acceptable salt thereof. Preferably, the pharmaceutical composition is in a unit dose form suitable for oral administration, such as a tablet or a capsule.

### EXAMPLES

The ability of the compounds of Formula I to selectively inhibit production of Aβ(1-42) was determined using the following assay:

### Cell-based γ-Secretase Assay

Human SH-SY5Y neuroblastoma cells overexpressing the direct γ-secretase substrate SPA4CT were induced with sodium butyrate (10 mM) for 4 hours prior to plating. Cells were plated at 35,000 cells/well/100 µl in 96-well plates in phenol red-free MEM/10% FBS, 50 mM HEPES, 1% Glutamine and incubated for 2 hrs at 37 °C, 5% CO₂.

Compounds for testing were diluted into Me₂SO to give a ten point dose-response curve. Typically 10 µl of these diluted compounds in Me₂SO were further diluted into 182 µl dilution buffer (phenol red-free MEM/10% FBS, 50 mM HEPES, 1% Glutamine) and 10 µl of each dilution was added to the cells in 96-well plates (yielding a final Me₂SO concentration of 0.5%). Appropriate vehicle and inhibitor controls were used to determine the window of the assay.

After incubation overnight at 37 °C, 5%CO₂, 10 µl and 50 µl media were transferred into a fresh Costar round-bottom 96-well plate for detection of Aβ(40) and Aβ(42) peptides, respectively. 40 µl Origen buffer (PBS, 2% BSA, 0.2% Tween-20) was added to the Aβ(40) wells followed by the addition of 25 µl the respective antibody premixes to the wells:
Aβ(40) premix: 1 µg/ml ruthenylated G2-10 antibody, 4 µg/ml biotinylated 4G8 antibody diluted in Origen buffer
Aβ(42) premix: 0.5 µg/ml ruthenylated G2-11 antibody, 4 µg/ml biotinylated 4G8 antibody diluted in Origen buffer

(Biotinylated 4G8 antibody supplied by Signet Pathology Ltd; G2-10 and G2-11 antibodies supplied by Chemicon)

After overnight incubation of the assay plates on a shaker at 4 °C, the Origen M8 Analyser (Igen Inc.) was calibrated according to the manufacturer's instructions. 25 µl of streptavidin magnetic bead (Dynal) premix (400 µg/ml streptavidin beads/ml in Origen buffer) was added to the assay plates and incubated on a shaker for 15 minutes. 150 µl Origen buffer was added to each well and the plates were read on the Origen M8 Analyser according to the manufacturer's instructions.

Cell viability was measured in the corresponding cells after removal of the media for the Aβ assays by a colorimetric cell proliferation assay (CellTiter 96™ AQ assay, Promega) utilizing the bioreduction of MTS (Owen's reagent) to formazan according to the manufacturer's instructions. Briefly, 5 µl of 10x MTS/PES was added to the remaining 50 µl of media before returning to the incubator. The optical density was read at 495 nm after -4 hours.

LD₅₀ and IC₅₀ values for inhibition of Aβ(40) and Aβ(42) were calculated by nonlinear regression fit analysis using the appropriate software (eg. Excel fit). The total signal and the background were defined by the corresponding Me₂SO and inhibitor controls.

The compounds listed in the following examples all gave IC₅₀ values for Aβ(1-42) inhibition that were at least 2-fold lower than the corresponding IC₅₀ values for Aβ(1-40) inhibition, typically at least 5-fold lower, and in the preferred cases at least 50-fold lower.

Representative IC₅₀ values for Aβ(1-42) inhibition obtained for compounds exemplified below were in the following ranges:
2.0-3.0µM - Examples 8, 12, 14, 23.
1.5-2.0µM - Examples 2, 7, 11, 15.
1.0-1.5µM - Examples 1, 6, 17, 22, 24.
0.5-1.0µM - Examples 3, 4, 5, 9, 10, 13, 16, 21
<0.5 µM - Examples 18, 19, 20.

### Assay for in vivo efficacy

APP-YAC transgenic mice (20-30 g; 2-6 months old) and Sprague Dawley rats (200-250 g; 8-10 weeks old) were kept on 12-hr light/dark cycle with unrestricted access to food and water. Mice and rats were fasted overnight and were then dosed orally at 10 ml/kg with test compound formulated in either imwitor:Tween-80 (50:50) or 10% Tween-80, respectively. For compound screening studies, test compounds were administered at a single dose (20 or 100 mg/kg) and blood was taken serially at 1 and 4 hrs via tail bleed from mice and terminally at 7 hrs for mice and rats via cardiac puncture. In dose response studies, compounds were given at 0.1, 3, 10, 30, and 100 mg/kg and blood was taken terminally at 7 hrs from mice and rats via cardiac puncture. Following euthanasia by CO₂, forebrain tissue was harvested from animals and stored at -80 degrees. For PD analysis of brain Aβ levels, soluble Aβ was extracted from hemi-forebrains by homogenization in 10 volumes of 0.2% DEA in 50 mM NaCl followed by ultracentrifugation. Levels of Aβ 42/40 were analyzed using Meso Scale technology (electrochemiluminesence) with biotinylated 4G8 capture antibody and ruthenium labeled 12F4 or G210 detection antibodies for Aβ 42 and Aβ 40, respectively. For PK analysis, blood and brain samples were processed using a protein precipitation procedure with the remaining filtrate being analyzed via LC/MS/MS to determine drug exposure levels, brain penetration, and ED50/EC50, where appropriate.

Reductions in Aβ42 levels (relative to vehicle-treated controls) for representative compounds of the invention were in the range 50-90% whereas corresponding reductions in Aβ40 levels for the same compounds were less than 20%.

### Example 1

### {1-{4,4-dimethyl-1-[4-(trifluoromethyl)phenyl]pentyl}-4,4-difluoro-2-[4-(trifluoromethyl)phenyl]piperidin-3-yl}acetic acid

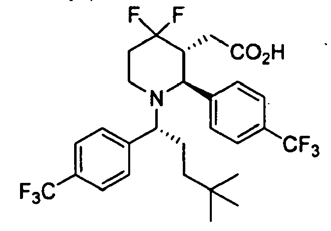

### Step 1: benzyl 4-oxo-2-[4-(trifluoromethyl)phenyl]-3,4-dihydropyridine-1(2H)-carboxylate

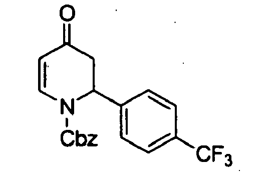

To a solution of 4-methoxypyridine (5g, 45.8 mmol) in THF (100 mL) was added benzyl chloroformate at 0 °C dropwise. The solution was stirred at this temperature for 1 hour then cooled to -78 °C. To this white, heterogeneous mixture was added 4-(trifluoromethyl)phenylmagnesium bromide (68.7 mL of a 1M solution, 68.7 mmol) and the reaction was warmed to 0 °C for 2 hours. The reaction was quenched with ammonium chloride solution, warmed to ambient temperature and partitioned between water/ethyl acetate. The organics were washed with brine, dried over sodium sulfate, filtered and evaporated *in vacuo.* The reaction was purified by flash column chromatography (20-40% EA/hexanes) to give 11.2g of benzyl 4-oxo-2-[4-(trifluoromethyl)phenyl]-3,4-dihydropyridine-1(2*H*)-carboxylate as a pale yellow solid. ¹H NMR (600 MHz, CDCl₃): δ 7.99 (bs, 1H), 7.54 (d, J = 8.2 Hz, 2H), 7.30 (m, 7H), 5.76 (bs, 1H), 5.41 (d, J = 7.9 Hz, 1H), 5.22 (dd, J = 40.2, 11.7 Hz, 2H), 3.19 (dd, J = 16.7, 7.9 Hz, 1H), 2.77 (d, J = 16.7 Hz, 1H); LC/MS (EIMS, M+H) = 376.1.

### Step 2: benzyl 3-(2-methoxy-2-oxoethyl)-4-oxo-2-[4-(trifluoromethyl)phenyl]-3,4-dihydropyridine-1(2H)-carboxylate

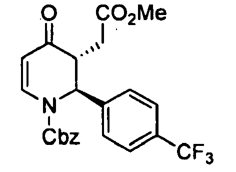

To a solution of benzyl 4-oxo-2-[4-(trifluoromethyl)phenyl]-3,4-dihydropyridine-1(2*H*)-carboxylate (3.56g, 9.48 mmol) in THF (50 mL) at -78 °C was added lithium (bis-trimethylsilyl)amide (10.43 mL of a 1M solution, 10.43 mmol) dropwise. The resulting solution was stirred at this temperature for 1 hour. The reaction was then treated with methyl bromoacetate (1.80 mL, 18.97 mmol) and slowly warmed to
0 °C. Upon stirring at this temperature for 1 hour the reaction was quenched with 1 M HCl solution and partitioned between water/ethyl acetate. The organics were washed with brine, dried over sodium sulfate, filtered and evaporated *in vacuo.* The reaction was purified by flash column chromatography (5-40% EA/hexanes) to give 2.55g of benzyl 3-(2-methoxy-2-oxoethyl)-4-oxo-2-[4-(trifluoromethyl)phenyl]-3,4-dihydropyridine-1(2*H*)-carboxylate as a clear oil. ¹H NMR (600 MHz, CDCl₃): δ8.07 (d, J = 7.6 Hz, 1H), 7.53 (d, J = 8.5 Hz, 2H), 7.30 (m, 7H), 5.69 (s, 1H), 5.38 (d, J = 8.5 Hz, 1H), 5.22 (m, 2H), 3.74 (s, 3H), 3.14 (dd, J = 10.3, 3.8 Hz, 1H), 2.65 (m, 2H); LC/MS (EIMS, M+H) = 448.2.

### Step 3: benzyl 3-(2-methoxy-2-oxoethyl)-4-oxo-2-[4-(trifluoromethyl)-phenyl]piperidine-1-carboxylate

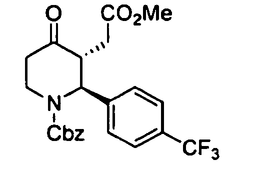

To a solution of benzyl 3-(2-methoxy-2-oxoethyl)-4-oxo-2-[4-(trifluoromethyl)phenyl]-3,4-dihydropyridine-1(2*H*)-carboxylate (0.8g, 1.79 mmol) in THF (10 mL) at -78 °C was added L-Selectride (2.53 mL of a 1M solution, 2.53 mmol) dropwise. The reaction was quenched with 1 mL concentrated ammonium chloride solution after 2 minutes. The reaction was warmed to ambient temperature and partitioned between water/ethyl acetate. The organics were washed with brine, dried over sodium sulfate, filtered and evaporated *in vacuo.* The reaction was purified by flash column chromatography (20% EA/hexanes) to give 700 mg of benzyl 3-(2-methoxy-2-oxoethyl)-4-oxo-2-[4-(trifluoromethyl)-phenyl]piperidine-1-carboxylate as a clear oil. ¹H NMR (600 MHz, CDCl₃): δ7.56 (d, J = 8.2 Hz, 2H), 7.30 (m, 7H), 5.08 (d, J = 12.3 Hz, 1H), 4.94 (m, 1H), 4.58 (m, 1H), 3.84 (m, 1H), 3.66 (bs, 1H), 3.57 (s, 3H), 3.45 (m, 1H), 3.62 (m, 2H), 2.21 (dd, J = 16.7,4.7 Hz, 1H), 0.92 (m, 1H); LC/MS (EIMS, M+H) = 450.2.

### Step 4: benzyl 4,4-difluoro-3-(2-methoxy-2-oxoethyl)-2-[4-(trifluoromethyl)-phenyl]piperidine-1-carboxylate

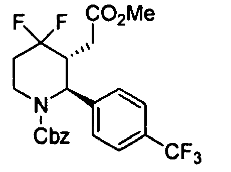

To a solution of benzyl 3-(2-methoxy-2-oxoethyl)-4-oxo-2-[4-(trifluoromethyl)-phenyl]piperidine-1-carboxylate (1.1g, 2.47 mmol) in methylene chloride (25 mL) at 0 °C was added [bis(2-methoxyethyl)amino]sulfur trifuoride (0.55 mL, 2.97 mmol). The reaction was allowed to warm to ambient temperature and stir for 16 hours. The reaction was quenched with ammonium chloride solution and partitioned between ethyl acetate/water. The organics were washed with brine, dried over sodium sulfate, filtered and evaporated *in vacuo.* The reaction was purified by flash column chromatography (20% EA/hexanes) to give 0.6g of benzyl 4,4-difluoro-3-(2-methoxy-2-oxoethyl)-2-[4-(trifluoromethyl)-phenyl]piperidine-1-carboxylate which was carried directly into the next reaction.

### Step 5: methyl {4,4-difluoro-2-[4-(trifluoromethyl)phenyl]piperidin-3-yl}acetate

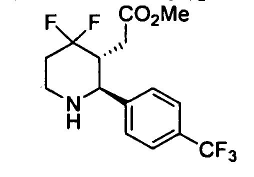

To a degassed solution of benzyl 4,4-difluoro-3-(2-methoxy-2-oxoethyl)-2-[4-(trifluoromethyl)-phenyl]piperidine-1-carboxylate (0.6g, 1.26 mmol) in methanol (8 mL) was added palladium hydroxide (20% on carbon, 89mg) and the solution was placed under an atmosphere of hydrogen for 45 minutes. The reaction mixture was degassed with nitrogen, filtered through celite (washing with methanol) and evaporated *in vacuo.* The reaction was purified by flash column chromatography (15-50% EA/hexanes w/1% ammonia) to give 0.37g of methyl {4,4-difluoro-2-[4-(trifluoromethyl)phenyl]piperidin-3-yl}acetate as a white solid. ¹H NMR (600 MHz, CDCl₃): δ7.58 (d, J = 8.2 Hz, 2H), 7.50 (d, J = 7.9 Hz, 2H), 3.63 (d, J = 10.6 Hz, 1H), 3.31 (s, 3H), 3.18 (m, 1H), 2.98 (td, J = 12.3, 2.6 Hz, 1H), 2.65 (m, 2H), 2.10 (m, 3H); LC/MS (EIMS, M+H) = 338.2.

### Step 6: methyl {1-{4,4-dimethyl-1-[4-(trifluoromethyl)phenyl]pent-2-yn-1-yl}-4,4-difluoro-2-[4-(trifluoromethyl)phenyl]piperidin-3-yl}acetate

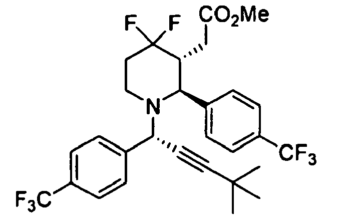

A solution of methyl {4,4-difluoro-2-[4-(trifluoromethyl)phenyl]piperidin-3-yl}acetate (120mg, 0.36 mmol), 4-(trifluoromethyl)benzaldehyde (0.1 mL, 0.75 mmol) and gold(III) bromide (15mg, 0.04 mmol) in water was degassed with nitrogen for 15 minutes. To this solution was added 3,3-dimethylbut-1-yne (0.09 mL, 0.73 mmol) and the reaction was sealed and heated to 75°C in the microwave for 12 hours. The reaction was diluted with ethyl acetate and filtered. The reaction was then partitioned between ethyl acetate and water. The organics were washed with brine, dried over sodium sulfate, filtered and evaporated *in vacuo.* The crude product was triturated with methanol and dried to give 65mg of methyl {1-{4,4-dimethyl-1-[4-(trifluoromethyl)phenyl]pent-2-yn-l-yl}-4,4-difluoro-2-[4-(trifluoromethyl)phenyl]piperidin-3-yl}acetate as a white solid. ¹H NMR (600 MHz, CDCl₃): δ7.64 (bs, 4H), 7.59 (d, J = 8.2 Hz, 2H), 7.55 (d, J = 8.2 Hz, 2H), 4.30 (s, 1H), 3.57 (d, J = 10.9 Hz, 1H), 3.31 (s, 3H), 2.87 (m, 1H), 2.64 (m, 2H), 2.43 (dd, J = 12.0, 2.3 Hz, 1H), 2.05 (m, 3H), 1.34 (d, J = 1.76 Hz, 9H); LC/MS (EIMS, M+H) = 576.2.

### Step 7: methyl {1-{4,4-dimethyl-1-[4-(trifluoromethyl)phenyl]pentyl}-4,4-difluoro-2-[4-(trifluoromethyl)phenyl]piperidin-3-yl}acetate

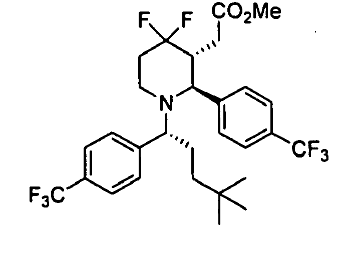

A solution of methyl {1-{4,4-dimethyl-1-[4-(trifluoromethyl)phenyl]pent-2-yn-1-yl}-4,4-difluoro-2-[4-(trifluoromethyl)phenyl]piperidin-3-yl}acetate (45mg, 0.08 mmol) in methanol (15 mL) was degassed with nitrogen and Raney nickel slurry was added (approx. 45mg of catalyst). The solution was placed on a Parr shaker under 50 psi hydrogen for 16 hours. The reaction was degassed with nitrogen and filtered through celite, washing catalyst with methylene chloride. The filtrate was evaporated *in vacuo* and carried into next reaction crude.

### Step 8: {1-{4,4-dimethyl-1-(4-(trifluoromethyl)phenyl)pentyl}-4,4-difluoro-2-(4-(trifluoromethyl)phenyl]piperidin-3-yl}acetic acid

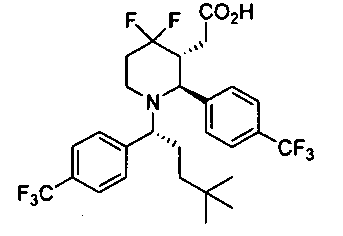

A solution of methyl {1-{4,4-dimethyl-1-[4-(trifluoromethyl)phenyl]pentyl}-4,4-difluoro-2-[4-(trifluoromethyl)phenyl]piperidin-3-yl} acetate (approx. 45mg, crude from step 7) and 1M potassium hydroxide in methanol (10 mL) was heated to 60 °C for 2 hours. The reaction was partitioned between 2M HCl and ethyl acetate. The organics were washed with brine, dried over sodium sulfate, filtered and evaporated *in vacuo.* The reaction was purified by reverse phase chromatography (20-100% acetonitrile/water) to give 20mg of {1-{4,4-dimethyl-1-[4-(trifluoromethyl)phenyl]pentyl}-4,4-difluoro-2-[4-(trifluoromethyl)phenyl]piperidin-3-yl}acetic acid as a white solid. ¹H NMR (600 MHz, CDCl₃): δ 7.76 (bs, 4H), 7.60 (d, J = 7.3 Hz, 2H), 7:31 (d, J = 7.3 Hz, 2H), 4.50 (bs, 1H), 4.17 (bs, 2H), 3.61 (bd, J = 9.4 Hz, 1H), 3.23 (bs, 2H), 2.90 (bm, 1H), 2.54 (d, J = 16.7 Hz, 1H), 2.18 (m, 2H), 1.89 (m, 1H), 1.78 (bm, 1H), 0.85 (s, 9H); LC/MS (EIMS, M+H) = 566.2.

### Examples 2-17

Examples 2-19 were made by the procedures in Example 1 using the piperidine from Step 5 and the appropriate aldehyde and acetylene in Step 6, omitting Step 7 in Examples 2, 3, 4, 7, 10, 13 and 17.

| Example | Structure | Name | M/Z |
|---|---|---|---|
| | | | ES⁺ |
| | | | [MH] + |
| 2 | 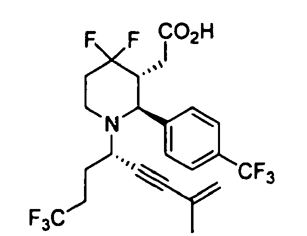 | {4,4-difluoro-1-[4-methyl-1-(3,3,3-trifluoropropyl)pent-4-en-2-yn-1-yl]-2-[4-(trifluoromethyl)phenyl]piperidin-3-yl}acetic acid | 498.2 |
| 3 | 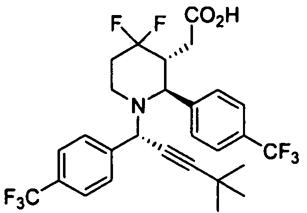 | {1-{4,4-dimethyl-1-[4-(trifluoromethyl)phenyl]pent-2-yn-1-yl}-4,4-difluoro-2-[4-(trifluoromethyl)phenyl]piperidin-3-yl}acetic acid | 562.2 |
| 4 | 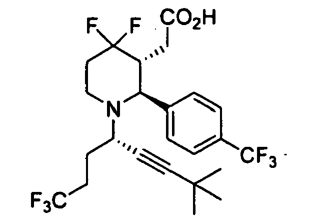 | {1-[4,4-dimethyl-1-(3,3,3-trifluoropropyl)pent-2-yn-1-yl]-4,4-difluoro-2-[4-(trifluoromethyl)phenyl]piperidin-3- yl}acetic acid | 514.2 |
| 5 | 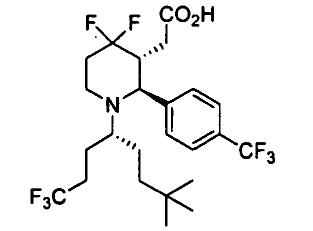 | {1-[4,4-dimethyl-1-(3,3,3-trifluoropropyl)pentyl]-4,4-difluoro-2-[4-(trifluoromethyl)phenyl]piperidin-3-yl}acetic acid | 518.2 |
| 6 | 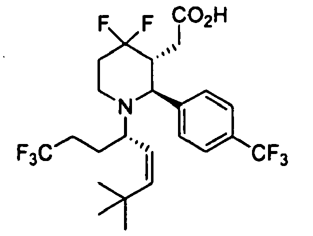 | {1-[(2Z)-4,4-dimethyl-1-(3,3,3-trifluoropropyl)pent-2-en-1-yl]-4,4-difluoro-2-[4-(trifluoromethyl)phenyl]piperidin-3-yl}acetic acid | 516.2 |
| 7 | 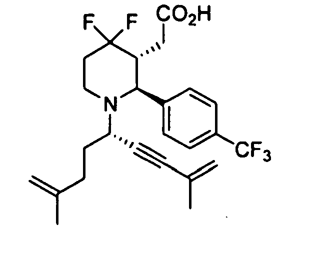 | {4,4-difluoro-1-[4-methyl-1-(3-methylbut-3-en-1-yl)pent-4-en-2-yn-1-yl]-2-[4-(trifluoromethyl)phenyl]piperidin-3-yl}acetic acid | 470.2 |
| 8 | 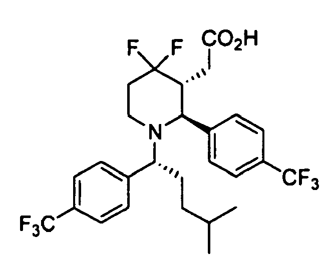 | {4,4-difluoro-1-(4-methyl-1-[4-(trifluoromethyl)phenyl]pentyl}-2-[4-(trifluoromenthyl)phenyl]piperidin-3-yl}acetic acid | 552.2 |
| 9 | 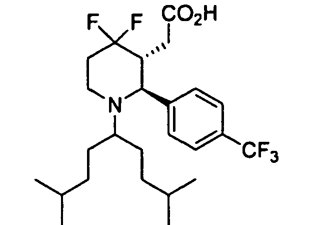 | {4,4-difluoro-1-(4-methyl-1-(3-methylbutyl)pentyl]-2-[4-(trifluoromenthyl)phenyl]piperidin-3-yl}acetic acid | 478.2 |
| 10 | 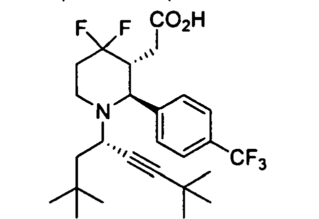 | {1-[1-(2,2-dimethylprpyl)-4,4-dimethylpent-2-yn-1-yl]-4,4-difluoro-2-}-2-[4-(trifluoromenthyl)phenyl]piperidin-3-yl}acetic acid | 488.2 |
| 11 | 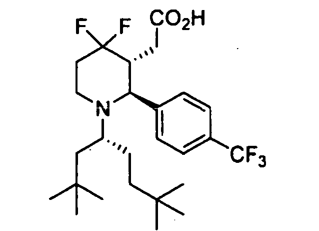 | {1-[2,2dimethylpropyl[4,4-dimethylpentyl]-4,4-difluoro-2-[4-(trifluoromenthyl)phenyl]-piperidin-3-yl}acetic acid | 492.2 |
| 12 | 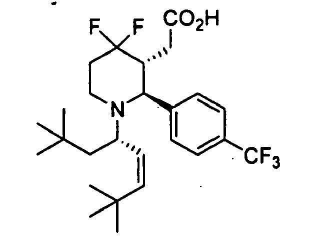 | {1-[(2Z)-1-(2,2-dimethylpropyl)-4,4-dimethylpent-2-en-1-yl]-4,4-difluoro-2-[4((trifluoromethyl)phenyl]piperidin-3-yl}acetic acid | 490.2 |
| 13 | 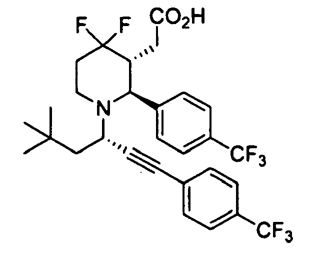 | {1-(3,3-dimethyl-1-{[4-(trifluoromethyl)pheny]ethyyl)butyk)-4,4-difluoro-2-[4-(trifluoromethyl)phenyl]piperidin-3-yl}acetic acid | 576.2 |
| 14 | 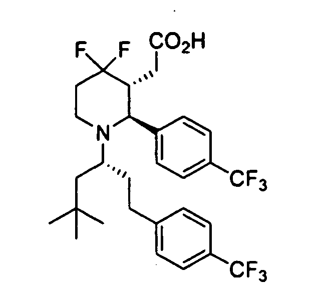 | {1-(3,3-dimethyl-1-{2-[4-(trifluoromethyl)phenyl]ethyl}butyl)-4,4-difluoro-2-[4-(trifluoromethyl)phenyl]piperidin-3-yl}acetic acid | 580.2 |
| 15 | 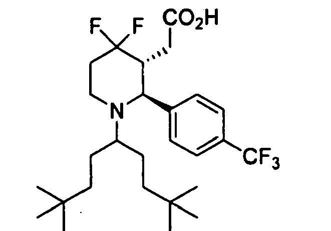 | {1-[1-(3,3-dimethylbutyl)-4,4-dimethylpentyl]-4,4-difluoro-2-[4-(trifluoromethyl)phenyl]piperidin-3-yl}acetic acid | 506.7 |
| 16 | 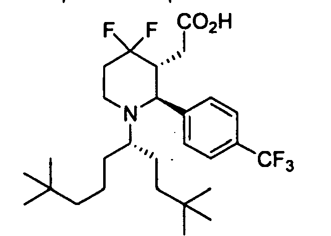 | {1-[1-(3,3-dimethylbutyl)-5,5-dimethylhexyl]-4,4-difluoro-2-[4-(trifluoromethyl)phenyl]piperidin-3-yl}acetic acid | 520.2 |
| 17 | 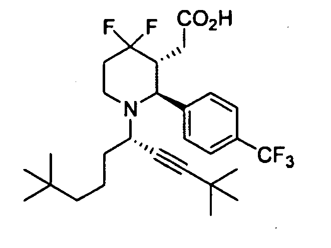 | {1-[1-(3,3-dimethylbut-1-yn-1-yl)-5,5-dimethylhexyl]-4,4-difluoro-2-[4-(trifluoromethyl)phenyl]piperidin-3-yl}acetic acid | 516.3 |

### Example 18

### {1-[4,4-dimethyl-1-(3,3,3-trifluoropropyl)pentyl]-4,4-difluoro-2-[4-(trifluoromethyl)phenyl]phenyl]piperidin-3-yl}acetic acid (single isomer)

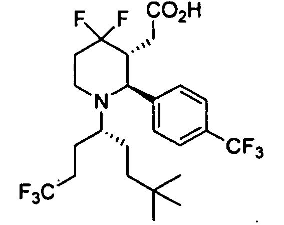

### Step 1: Separation of enantiomers of methyl {4,4-difluoro-2-[4-(trifluoromethyl)phenyl]piperidin-3-yl}acetate

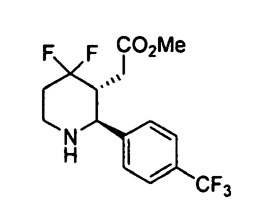

Methyl {4,4-difluoro-2-[4-(trifluoromethyl)phenyl]piperidin-3-yl}acetate (prepared in step 5, example 1) was purified by chiral chromatography with a Chiracel AD column (7.5% ethanol/heptane) to give the two pure enantiomers. The faster eluting enantiomer was carried on to final product.

### Step 2: methyl {1-[4,4-dimethyl-1-(3,3,3-trifluoropropyl)pent-2-yn-1-yl]-4,4-difluoro-2-[4-(trifluoromethyl)phenyl]piperidin-3-yl}acetate

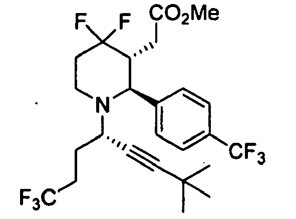

A solution of methyl {4,4-difluoro-2-[4-(trifluoromethyl)phenyl]piperidin-3-yl}acetate (150mg, 0.45 mmol), 4,4,4-trifluorobutanal (0.17 mg, 1.34 mmol) and gold(III) bromide (19mg, 0.045 mmol) in water (1.5 mL) was degassed with nitrogen for 15 minutes. To this solution was added 3,3-dimethylbut-1-yne (0.16 mL, 1.34 mmol) and the reaction was sealed and heated to 75°C in the microwave for 3 hours. The reaction was diluted with dichloromethane. The dichloromethane layer was loaded directly onto a silica column and the product was purified by flash column chromatography (0-5% ethyl acetate/hexanes) to give 215mg of methyl {(2*S*,3*R*)-1-[(1*S*)-4,4-dimethyl-1-(3,3,3-trifluoropropyl)pent-2-yn-1-yl]-4,4-difluoro-2-[4-(trifluoromethyl)phenyl]piperidin-3-yl}acetate as a clear oil. LC/MS (EIMS, M+H) = 528.1.

### Step 3: methyl {1-[4,4-dimethyl-1-(3,3,3-trifluoropropyl)pentyl]-4,4-difluoro-2-[4-(trifluoromethyl)phenyl]piperidin-3-yl}acetate

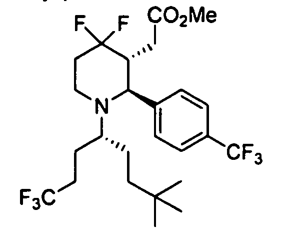

A solution of methyl {(2*S*,3*R*)-1-[(1*S*)-4,4-dimethyl-1-(3,3,3-trifluoropropyl)pent-2-yn-1-yl]-4,4-difluoro-2-[4-(trifluoromethyl)phenyl]piperidin-3-yl}acetate (80mg, 0.15 mmol) in methanol (15 mL) was degassed with nitrogen and Raney nickel slurry was added (approx. 45mg of catalyst). The solution was placed on a Parr shaker under 50 psi hydrogen for 16 hours. The reaction was degassed with nitrogen and filtered through celite, washing catalyst with methylene chloride. The filtrate was evaporated *in vacuo* and carried into next reaction crude.

### Step 4: {1-[4,4-dimethyl-1-(3,3,3-trifluoropropyl)pentyl]-4,4-difluoro-2-[4-(trifluoromethyl)phenyl]piperidin-3-yl}acetic acid

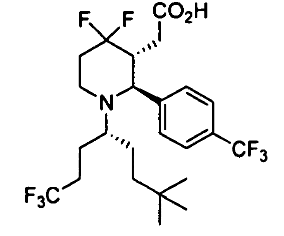

A solution of methyl {(2*S*,3*R*)-1-[(1*R*)-4,4-dimethyl-1-(3,3,3-trifluoropropyl)pentyl]-4,4-difluoro-2-[4-(trifluoromethyl)phenyl]piperidin-3-yl}acetate (approx. 45mg, crude from step 3) and 1M potassium hydroxide in methanol (2 mL) was heated to 60 °C for 16 hours. The reaction was partitioned between 2M HCl and ethyl acetate. The organics were washed with brine, dried over sodium sulfate, filtered and evaporated *in vacuo.* The reaction was purified by reverse phase chromatography (35-100% acetonitrile/water) to give {1-[4,4-dimethyl-1-(3,3,3-trifluoropropyl)pentyl]-4,4-difluoro-2-[4-(trifluoromethyl)phenyl]piperidin-3-yl}acetic acid as a white solid. ¹H NMR (600 MHz, CD₃OD: δ7.68 (d, J = 7.33 Hz, 2H), 7.55 (bs, 2H), 3.70 (bs, 1H), 3.08 (bs, 1H), 2.85 (bs, 1H), 2.60 (bs, 1H), 2.54 (dd, J = 16.4, 5.0 Hz, 1H), 2.44 (m, 1H), 2.20 (bm, 3H), 1.89 (dd, J = 16.7, 4.7 Hz, 2H), 1.60 (bm, 3H), 1.10 (bs, 1H), 1.00 (td, J = 12.9, 4.7 Hz, 1H), 0.85 (s, 9H), 0.57 (t, J = 10.6 Hz, 1H); LC/MS (EIMS, M+H) = 518.2.

### Examples 19-22, 22A

Examples 19-22 and 22A were made by the procedures in Example 18 using the enantiopure piperidine from Step 1 and the appropriate aldehyde and acetylene, omitting the hydrogenation step in Examples 19, 21 and 22A.

| Example | Structure | Name | M/Z ES⁺ [MH]⁺ |
|---|---|---|---|
| 19 | 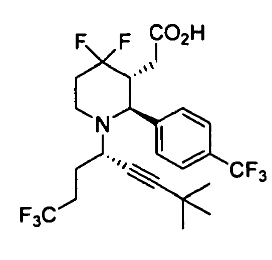 | {1-[4,4-dimethyl-1-(3,3,3-trifluoropropyl)pent-2-yn-1-yl]-4,4-difluoro-2-[4-(trifluoromethyl)phenyl]piperidin-3-yl}acetic acid | 514.2 |
| 20 | 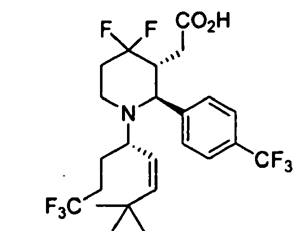 | {1-[(2*Z*)-4,4-dimethyl-1-(3,3,3-trifluoropropyl)pent-2-en-1-yl]-4,4-difluoro-2-[4-(trifluoromethyl)phenyl]piperidin-3-yl}acetic acid | 516.2 |
| 21 | 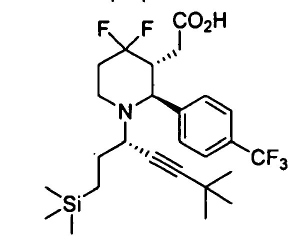 | {1-{4,4-dimethyl-1-[2-(trimethylsilyl)ethyl]pent-2-yn-1-yl}-4,4-difluoro-2-[4-(trifluoromethyl)phenyl]piperidin-3-yl}acetic acid | 518.2 |
| 22 | 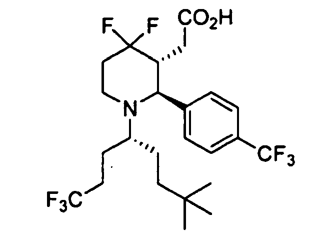 | {1-[4,4-dimethyl-1-(3,3,3-trifluoropropyl)pentyl]-4,4-difluoro-2-[4-(trifluoromethyl)phenyl]piperidin-3-yl}acetic acid | 534.2 |
| 22A | 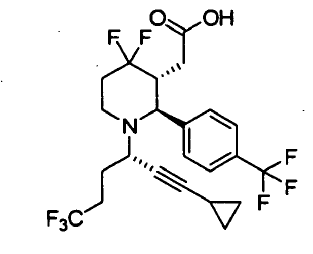 | {1-[1-(cyclopropylethynyl)-4,4,4-trifluorobutyl]-4,4-difluoro-2-[4-(trifluoromethyl)phenyl]piperidin-3-yl}acetic acid | 498.2 |

NB - Examples 18 - 22 and 22A are enantiopure compounds in which the relative configurations of the chiral atoms are as indicated in the structural formulae, but the absolute configurations have not been determined.

### Example 23

### {1-(4,4-dimethylpent-2-yn-1-yl)-4,4-difluoro-2,6-bis[4-(trifluoro-methyl)phenyl]piperidin-3-yl}acetic acid

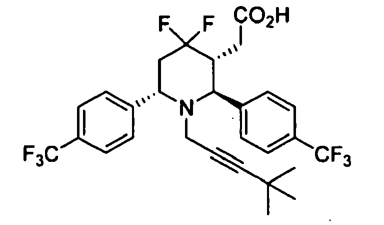

### Sten 1: benzyl 3-(2-tert-butoxy-2-oxoethyl)-4-oxo-2-[4-(trifluoromethyl)phenyl]-3,4-dihydropyridine-1(2H)-carboxylate

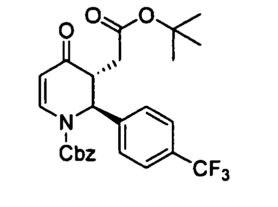

Prepared by same procedure outlined in Example 1, Step 2 using *tert*-butyl bromoacetate.

### Step 2: benzyl 3-(2-tert-butoxy-2-oxoethyl)4-oxo-2,6-bis[4-(trifluoro-methyl)phenyl]piperidine-1-carboxylate

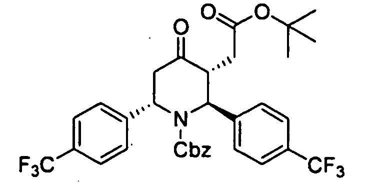

A solution of 4-(trifluormethyl)-phenylmagnesium bromide (1.9 mL of a 1M solution, 1.9 mmol) and copper iodide (52mg, 0.27 mmol) in THF (15 mL) was stirred at 0 °C for 1 hour. To this solution was added benzyl 3-(2-*tert*-butoxy-2-oxoethyl)-4-oxo-2-[4-(trifluoromethyl)phenyl]-3,4-dihydropyridine-1(2*H*)-carboxylate (0.53g, 1.08 mmol) as a solution in THF (10 mL) at 0 °C. After 1 hour the reaction was quenched with 10% ammonium hydroxide/concentrated ammonium chloride solution. The reaction was partitioned between water/ether and the organics were dried over sodium sulfate, filtered and evaporated *in vacuo.* The reaction was purified by flash column chromatography (5-25% EA/hexanes) to give benzyl 3-(2-*tert*-butoxy-2-oxoethyl)-4-oxo-2,6-bis[4-(trifluoro-methyl)phenyl]piperidine-1-carboxylate. ¹H NMR (600 MHz, CDCl₃): δ7.63 (d, J = 8.2 Hz, 2H), 7.55 (m, 4H), 7.23 (m, 3H), 6.87 (d, J = 6.7 Hz, 2H), 5.71 (s, 1H), 5.66 (d, J = 7.0 Hz, 1H), 4.98 (dd, J = 68.9 Hz, 12.3 Hz, 2H), 3.45 (m, 1H), 3.12 (dd, J = 14.7, 7.9 Hz, 1H), 2.92 (dd, J = 17.0, 3.2 Hz, 1H), 2.61 (m, 1H), 1.48 (s, 9H); LC/MS (EIMS, M+Na) = 658.0.

### Step 3: benzyl 3-(2-tert-butoxy-2-oxoethyl)-4,4-difluoro-2,6-bis[4-(trifluoromethyl)phenyl]piperidine-1-carboxylate

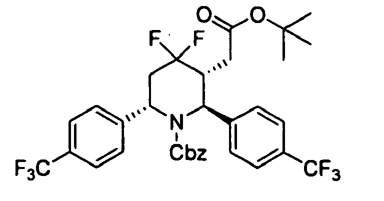

To a solution of benzyl 3-(2-*tert*-butoxy-2-oxoethyl)-4-oxo-2,6-bis[4-(trifluoromethyl)phenyl]piperidine-1-carboxylate (0.25g, 0.39 mmol) in methylene chloride (2 mL) at 0 °C was added [bis(2-methoxyethyl)amino]sulfur trifluoride (0.22 mL, 1.18 mmol) and the solution was warmed to ambient temperature and stirred for 16 hours. The reaction was quenched with ammonium chloride solution and partitioned between water/ethyl acetate. The organics were washed with brine, dried over sodium sulfate, filtered and evaporated *in vacuo.* The reaction was purified by flash column chromatography (5-15% ethyl acetate/hexanes) to give benzyl 3-(2-*tert-*butoxy-2-oxoethyl)-4,4-difluoro-2,6-bis[4-(trifluoromethyl)phenyl]piperidine-1-carboxylate, which was carried directly into the next reaction.

### Step 4: tert-butyl {4,4-difluoro-2,6-bis[4-(trifluoromethyl)phenyl]piperidin-3-yl}acetate

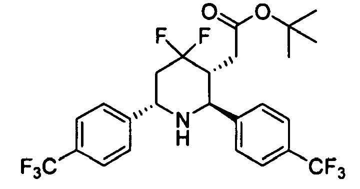

A solution of benzyl 3-(2-*tert*-butoxy-2-oxoethyl)-4,4-difluoro-2,6-bis[4-(trifluoromethyl)phenyl]piperidine-1-carboxylate (26 mg, 0.04 mmol) in methanol (100 mL) was degassed with nitrogen and treated with palladium hydroxide (approx. 20 mg, 20% on carbon) and placed under a hydrogen atmosphere for 45 minutes. The reaction was degassed with nitrogen, filtered through celite (washing with methanol) and evaporated *in vacuo.* The reaction was purified by flash column chromatography (5-20% ethyl acetate/hexanes) to give *tert*-butyl {4,4-difluoro-2,6-bis[4-(trifluoromethyl)-phenyl]piperidin-3-yl}acetate. LC/MS (EIMS, M+H) = 524.1.

### Step 5: tert-butyl {1-(4,4-dimethylpent-2-yn-1-yl)-4,4-difluoro-2,6-bis[4-(trifluoromethyl)phenyl]piperidin-3-yl}acetate

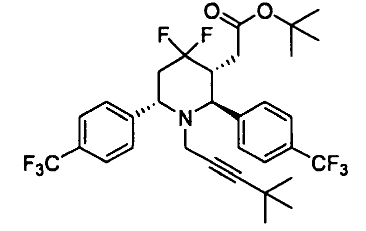

A solution of *tert*-butyl {4,4-difluoro-2,6-bis[4-(trifluoromethyl)-phenyl]piperidin-3-yl}acetate (27mg, 0.052 mmol), formaldehyde (1 mL, 37% aqueous solution) and gold(III) bromide (3mg, 0.005 mmol) in water was degassed with nitrogen for 15 minutes. To this solution was added 3,3-dimethylbut-1-yne (0.018 mL, 0.16 mmol) and the reaction was sealed and heated to 75°C in the microwave for 3 hours. The reaction was diluted with methylene chloride and filtered. The organics were washed with brine, dried over sodium sulfate, filtered and evaporated *in vacuo.* The reaction was purified by flash chromatography (0-5% ethyl acetate/hexanes) to give *tert-*butyl {1-(4,4-dimethylpent-2-yn-1-yl)-4,4-difluoro-2,6-bis[4-(trifluoromethyl)phenyl]piperidin-3-yl}acetate semi-pure. LC/MS (EIMS, M+H) = 618.2.

### Step 6: {1-(4,4-dimethylpent-2-yn-1-yl)-4,4-difluoro-2,6-bis[4-(trifluoromethyl)phenyl]piperidin-3-yl}acetic acid

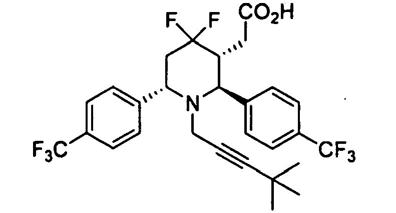

A solution of *tert*-butyl {1-(4,4-dimethylpent-2-yn-1-yl)-4,4-difluoro-2,6-bis[4-(trifluoromethyl)phenyl]piperidin-3-yl}acetate (10mg, 0.016 mmol) in methylene chloride (2 mL) was treated with trifluoroacetic acid (0.5 mL) and stirred at ambient temperature for 1 hour. The reaction was evaporated *in vacuo* and purified by reverse phase chromatography to give {1-(4,4-dimethylpent-2-yn-1-yl)-4,4-difluoro-2,6-bis[4-(trifluoromethyl)phenyl]piperidin-3-yl}acetic acid as a white solid. ¹H NMR(600 MHz, CDCl₃): δ7.64 (m, 8H), 4.78 (m, 1H), 4.42 (d, J = 7.3 Hz, 1H), 3.28 (m, 1H), 3.20 (d, J = 17.0 Hz, 1H), 3.12 (d, J = 17.0 Hz, 1H), 2.65 (m, 1H), 2.64 (dd, J = 17.6, 5.3 Hz, 1H), 2.58 (m, 1H), 2.40 (dd, J = 17.3, 6.2 Hz, 1H), 1.15 (s, 9H); LC/MS (EIMS, M+H) = 562.2.

### Example 24

### {1-(4,4-dimethylpentyl)-4,4-difluoro-2,6-bis[4-(trifluoromethyl)phenyl]piperidin-3-yl}acetic acid

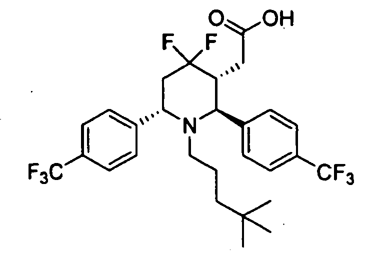

### Step 1: tert-butyl {1-(4,4-dimethylpentyl)-4,4-difluoro-2,6-bis[4-(trifluoromethyl)phenyl]piperidin-3-yl}acetate

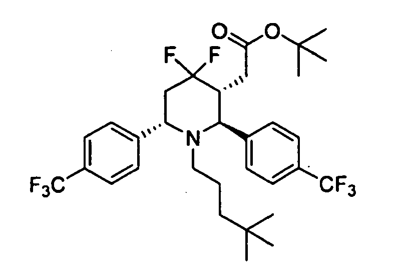

A solution of *tert*-butyl {1-(4,4-dimethylpent-2-yn-1-yl)-4,4-difluoro-2,6-bis[4-(trifluoromethyl)phenyl]piperidin-3-yl}acetate (22mg, 0.036 mmol) in methanol (15 mL) was degassed with nitrogen and treated with Raney nickel (approx. 25mg). The solution was placed under 50 psi hydrogen on a Parr shaker for 48 hours. The reaction was filtered through celite (washing with methylene chloride) and evaporated *in vacuo* to give *tert*-butyl {1-(4,4-dimethylpentyl)-4,4-difluoro-2,6-bis[4-(trifluoromethyl)phenyl]-piperidin-3-yl}acetate. LC/MS (EIMS, M+H) = 622.2.

### Step 2: {1-(4,4-dimethylpentyl)-4,4-difluoro-2,6-bis[4-(trifluoromethyl)phenyl]-piperidin-3-yl}acetic acid

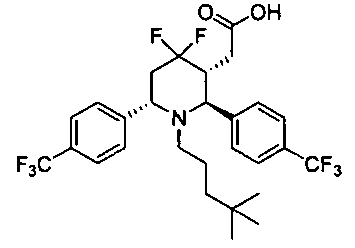

A solution of *tert*-butyl {1-(4,4-dimethylpentyl)-4,4-difluoro-2,6-bis[4-(trifluoromethyl)phenyl]piperidin-3-yl} acetate (22mg, 0.036 mmol) in methylene chloride (1 mL) was treated with trifluoroacetic acid (1 mL) and stirred for 1 hour at ambient temperature. The reaction was evaporated *in vacuo* and purified by reverse phase chromatography to give {1-(4,4-dimethylpentyl)-4,4-difluoro-2,6-bis[4-(trifluoromethyl)phenyl]-piperidin-3-yl}acetic acid as a white solid. ¹H NMR (600 MHz, CDCl₃): δ7.66 (m, 8H), 4.87 (bs, 1H), 4.60 (bs, 1H), 3.18 (bs, 1H), 2.50 (bm, 6H), 1.18 (bs, 2H), 0.68 (s, 9H); LC/MS (EIMS, M+H) = 566.2.

### Example 25

### {(2R,4S)-1-[(1R)-4,4-dimethyl-1-(3,3,3-trifluoropropyl)pentyl]-3,3-difluoro-2-[4-(trifluoromethyl)phenyl]piperidin-4-yl}acetic acid

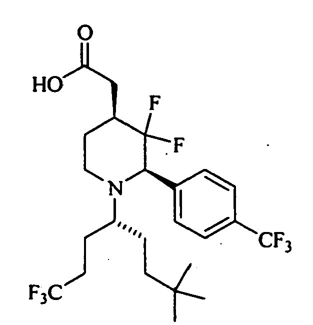

### Step 1: methyl {(2S,4R)-2-[4-(trifluoromethyl)phenyl]piperidin-4-yl} acetate

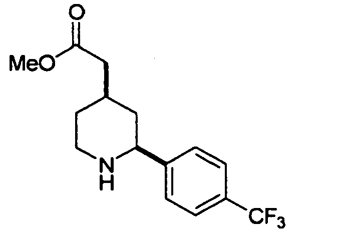

- prepared as described in WO 2006/043064 (example 114 step 1).

### Step 2: methyl {(2S,4R)-1-[(1S)-4,4-dimethyl-1-(3,3,3-trifluoropropyl)pent-2-yn-1-yl]-2-[4-(trifluoromethyl)phenyl]piperidin-4-yl}acetate

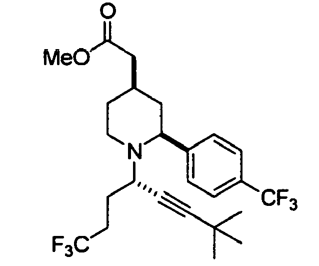

The product of Step 1 (5.00 g, 16.59 mmol), 3,3-dimethyl-1-butyne (4.09 ml, 33.2 mmol), AuBr₃ (0.362 g, 0.830 mmol), 4,4,4-trifluorobutyraldehyde (3.14 g, 24.89 mmol) and water (30 ml) were heated in an oil bath at 75 °C for 1 h. The mixture was cooled and 10 mL of CH₂Cl₂ was added. The organic fraction was purified by column chromatography on silica gel Biotage 40M, eluting with ethyl ether/hexanes to give title compound (5.39 g, 10.97 mmol, 66.1 % yield) as a yellow oil. MS cal'd 492 (MH⁺), exp 492 (MH⁺).

### Step 3: methyl {(2S,4R)-1-[(1R)-4,4-dimethyl-1-(3,3,3-trifluoropropyl)pentyl]-2-[4-(trifluoromethyl)phenyl]piperidin-4-yl}acetate

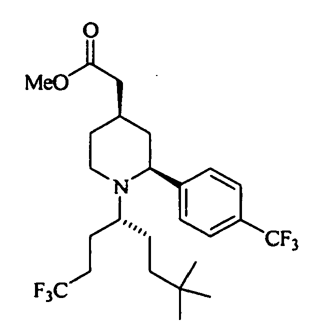

The product of Step 2 (5.39 g, 10.97 mmol) was dissolved in EtOH (50 ml) and the solution was purged with N₂. Palladium on carbon (10%) (5.84 g, 5.48 mmol) was added and the flask was evacuated and purged 3 times with hydrogen then pressurized with a hydrogen balloon. After 4 h the mixture was transferred to a Parr shaker and pressurized to 50 psi. After 72 h the flask was purged with nitrogen, the solution was filtered and concentrated to give an oil. The residue was purified by column chromatography on silica gel Biotage 40M, eluting with CH₂Cl₂/hexanes to give the title compound (2.65 g, 5.35 mmol, 48.8 % yield) as a colorless oil. MS cal'd 496 (MH⁺), exp 496 (MH⁺).

### Step 4: methyl {(2S,4S)-1-[(1R)-4,4-dimethyl-1-(3,3,3-trifluoropropyl)pentyl]-3,3-difluoro-2-hydroxy-2-[4-(trifluoromethyl)phenyl]piperidin-4-yl}acetate

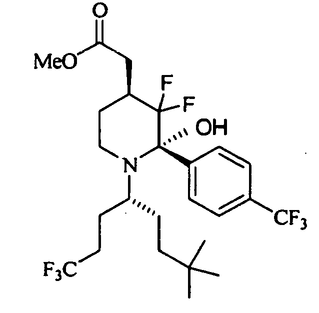

The product of Step 3 (296 mg, 0.597 mmol) and Selectfluor™ (677 mg, 1.911 mmol) were dissolved in DMF (2987 µl) and stirred for 1 h. The mixture was cooled, water (30 mL) was added and the mixture was extracted with diethyl ether (100 mL). The organic fraction was washed with brine (30 mL), dried (MgSO₄), filtered and the solvent was evaporated under reduced pressure. The residue was purified by column chromatography on silica gel Biotage 25S, eluting with ethyl ether/hexanes to give the title compound as a colorless oil. MS cal'd 548 (MH⁺), exp 548 (MH⁺).

### Step 5: methyl {(2R,4S)-1-[(1R)-4,4-dimethyl-1-(3,3,3-trifluoropropyl)pentyl]-3,3-difluoro-2-[4-(trifluoromethyl)phenyl]piperidin-4-yl}acetate

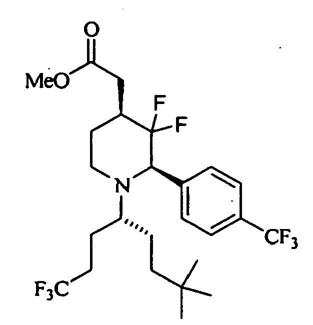

Borane THF complex (0.747 ml, 0.747 mmol) was added to a stirred, cooled -78 °C mixture of the product of Step 4 and THF. The mixture was stirred at -78 °C for 30 min then warmed to room temperature over 1h then stirred at that temperature for 1 h. Aqueous ammonium chloride (saturated, 30 mL) was added and the mixture was extracted with dichloromethane (70 mL). The organic fraction was washed with brine, dried (Na₂SO₄), filtered and the solvent was evaporated under reduced pressure. The residue was purified by column chromatography on silica gel Biotage 25S, eluting with ether/hexanes to give 181 mg (60%) of the title compound as a colorless oil. MS cal'd 532 (MH⁺), exp 532 (MH⁺).

### Step 6: {(2R,4S)-1-[(1R)-4,4-dimethyl-1-(3,3,3-trifluoropropyl)pentyl]-3,3-difluoro-2-[4-(trifluoromethyl)phenyl]piperidin-4-yl}acetic acid

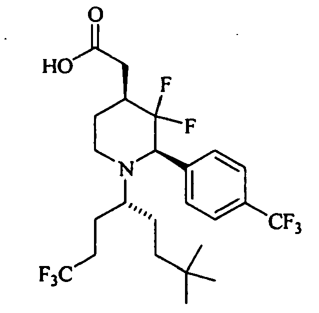

The product of Step 5 (108 mg, 0.203 mmol) and LiOH 2 (9.73 mg, 0.406 mmol) were dissolved in THF, water and MeOH and stirred for 18 h. Trifluoroacetic acid (47.0 µL, 0.610 mmol) was added, and the solvent was removed under reduced pressure. The residue was purified by preparative HPLC Reverse phase (C-18), eluting with acetonitrile/water + 0.05% TFA to give 55 mg (43%) of title compound as a white solid. ¹H NMR (600 MHz, CD₃OD) δ 0.49 (sextet, *J* = 7.1), 0.83 (m, 10), 0.98 (m, 1), 1.06 (m, 1), 1.59 (qd, 4, *J* = 11.8, 4.2), 1.98 (m, 2), 2.23 (m, 2), 2.45 (m, 3), 2.71 (dd, 1, *J* = 16.3, 3.9), 3.02 (d, 1, *J* = 11.4), 4.01 (d, 1, *J* = 21.3), 7.58 (br s, 2), 7.67 (d, 1, *J* = 6.8). MS cal'd 532 (MH⁺), exp 532 (MH⁺).

### Example 26

### ((2R,4S)-3,3-difluoro-2-[4-(trifluoromethyl)phenyl]-1-{(1S)-4,4,4-trifluoro-1-[2-(trimethylsilyl)ethyl]butyl}piperidin-4-yl)acetic acid

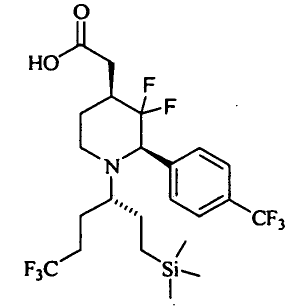

- prepared in a similar manner to Example 25, substituting trimethylsilylacetylene for 3,3-dimethylbut-1-yne in Step 1. The product was purified by preparative HPLC Reverse phase (C-18), eluting with acetonitrile/water + 0.05% TFA to give the title compound as a white solid. NMR (600 MHz, CD₃OD) δ -0.19 (m, 1), -0.05 (s, 9), 0.32 (sextet, 1, *J* = 7.5), 1.14 (m, 1), 1.62 (m, 2), 1.71 (m, 1), 2.00 (m, 2), 2.26 (dt, 2, *J* = 16.4, 8.2), 2.46 (m, 2), 2.55 (m, 1), 2.71 (dd, 1, *J* = 16.4, 4.0), 3.09 (d, 1, *J* = 11.7), 4.15 (d, 1, *J =* 21.3), 7.59 (br s, 2), 7.60 (d, 1, *J* = 7.6). MS cal'd 534 (MH⁺), exp 534 (MH⁺).

### Example 27

### {(2R,4S)-3,3-difluoro-1-{(1R)-4-methyl-1-[4-(trifluoromethyl)phenyl]pentyl}-2-[4-(trifluoromethyl)phenyl]piperidin4-yl}acetic acid

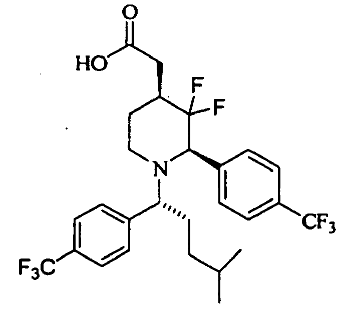

- prepared from methyl {(2*S*,4*R*)-1-{(1*R*)-4-methyl-1-[4-(trifluoromethyl)phenyl]pentyl}-2-[4-(trifluoromethyl)phenyl]piperidin-4-yl}acetate (WO 2006/043064 Example 114 step 2) by fluorination, reduction and hydrolysis using the procedures described in Example 25 Steps 4, 5 and 6. The crude material was found to be 96% pure by LCMS. ¹H NMR (600 MHz, CD₃OD) δ 0.51-0.57 (m, 1), 0.78 (d, 3, *J* = 6.7), 0.79 (d, 3, *J* = 6.7), 0.81-0.88 (m, 1), 1.34-1.42 (m, 1), 1.48-1.55 (m, 1), 1.88-1.93 (m, 1), 1.99-2.07 (m, 2), 2.23 (dd, 1, *J* = 16.6, 9.4), 2.54-2.60 (m, 1), 2.71 (dd, 1, *J* = 16.5, 3.9), 2.88 (t, 1, *J* = 11.8), 3.02 (d, 1, *J* = 11.4), 3.70 (d, 1, *J* = 11.3), 4.55 (d, *1, J* = 22.1), 7.53 (d, 2, *J* = 8.2), 7.68 (d, 2, *J* = 8.2), 7.82 (s, 4). MS cal'd 552 (MH⁺), exp 552 (MH⁺).

## Claims

1. A compound of formula I: 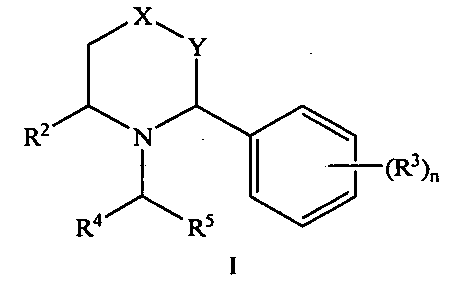 or a pharmaceutically acceptable salt or hydrate thereof; wherein:
n is 0, 1, 2 nor 3;
one of X and Y represents CF₂ and the other represents CH-C(R¹)₂-Z;
Z represents CO₂H or a tetrazole ring;
each R¹ independently represents H or a non-aromatic hydrocarbon group of up to 6 carbon atoms; or the two R¹ groups complete a C₃₋₆alicyclic group;
R² represents H or phenyl which optionally bears up to 3 substituents independently selected from halogen, C₁₋₆alkyl bearing 0-3 fluorine substituents, C₁₋₆alkoxy bearing 0-3 fluorine substituents, and C₂₋₆alkenyl;
each R³ independently represents halogen, C₁₋₆alkyl bearing 0-3 fluorine substituents, C₁₋₆alkoxy bearing 0-3 fluorine substituents, or C₂₋₆alkenyl; and
R⁴ and R⁵ independently represent H or hydrocarbon of up to 12 carbon atoms which optionally bears up to 3 substituents selected from halogen, perfluoroC₁₋₄alkyl, CN, Si(C₁₋₄alkyl)₃, OH, C₁₋₄alkoxy and OCF₃.

2. A compound according to claim 1 wherein Z represents CO₂H.

3. A compound according to claim 1 wherein R² represents H or phenyl which is substituted with 2-CF₃, 3-CF₃, 4-CF₃, 2,4-di(CF₃), 2-F-4-CF₃, 4-OCF₃, 4-allyl, 4-n-propyl, 4-isopropropyl or 4-tert-butyl.

4. A compound according to claim 3 wherein R² represents H or 4-trifluoromethylphenyl.

5. A compound according to claim 1 wherein (R³)ₙ represents 2-CF₃, 3-CF₃, 4-CF₃, 2,4-di(CF₃), 2-F-4-CF₃, 4-OCF₃, 4-allyl, 4-n-propyl, 4-isopropyl or 4-tert-butyl.

6. A compound according to claim 1 wherein R⁴ and R⁵ are independently selected from linear or branched C₁₋₁₂alkyl, alkenyl and alkynyl groups, C₃₋₆alicyclic groups, C₃₋₆alicyclicC_{1- 6}alkyl groups, phenyl groups, phenylC₁₋₆alkyl groups, phenylC₂₋₄alkynyl groups and C₃₋₆alicyclicC₂₋₄alkynyl groups and are optionally substituted with halogen, C₁₋₄perfluoroalkyl or Si(C₁₋₄alkyl)₃.

7. A compound according to claim 1 which is a compound of formula II: 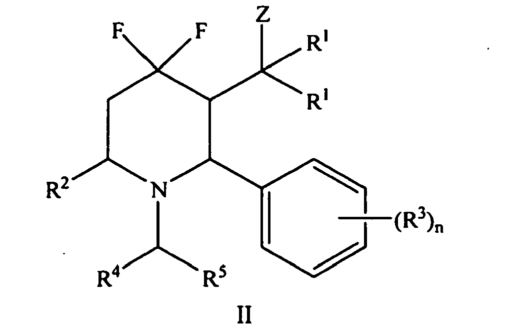 or a pharmaceutically acceptable salt or hydrate thereof;
wherein n, Z, R¹, R², R³, R⁴ and R⁵ are as defined in claim 1.

8. A compound according to claim 7 wherein the relative stereochemical configurations of the substituents on the piperidine ring are as shown in formula IIA:

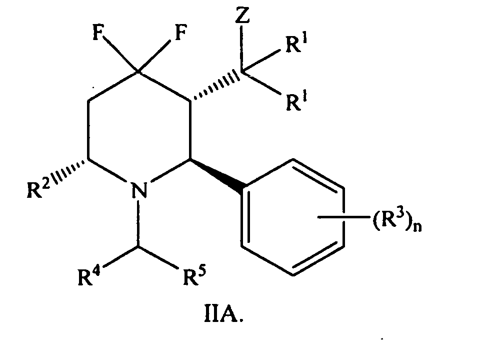

9. A compound according to claim 1 which is a compound of formula III: 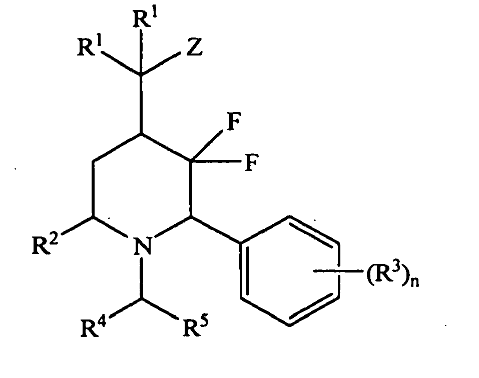 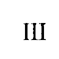 or a pharmaceutically acceptable salt or hydrate thereof;
wherein n, Z, R¹, R², R³, R⁴ and R⁵ are as defined in claim 1.

10. A compound according to claim 9 wherein the relative stereochemical configurations of the substituents on the piperidine ring are as shown in formula IIIA:

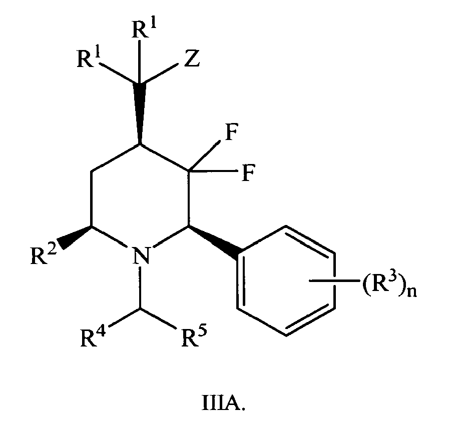

11. A pharmaceutical composition comprising a compound according to any previous claim and a pharmaceutically acceptable carrier.

12. A compound according to any of claims 1-10 for use in therapeutic treatment of the human body.

13. A compound according to claim 12 which is for use in treatment or prevention of a disease associated with deposition of β-amyloid in the brain.

14. A compound according to claim 13 which is for use in treatment or prevention of Alzheimer's disease.

## Patentansprüche

1. Eine Verbindung der Formel I: 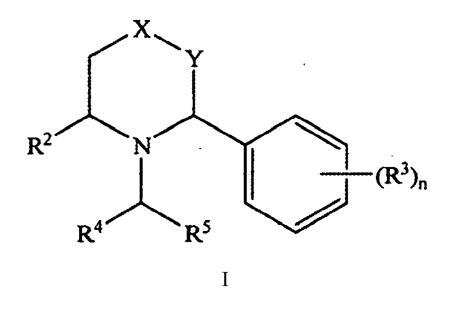 oder ein pharmazeutisch annehmbares Salz oder Hydrat davon, wobei:
n 0, 1, 2 oder 3 ist,
eines von X und Y CF₂ bedeutet und das andere CH-C(R¹)₂-Z bedeutet,
Z CO₂H oder einen Tetrazolring bedeutet,
jedes R¹ unabhängig H oder eine nichtaromatische Kohlenwasserstoffgruppe mit bis zu 6 Kohlenstoffatomen bedeutet, oder die beiden R¹-Gruppen eine C₃₋₆-alicyclische Gruppe vervollständigen,
R² H oder Phenyl bedeutet, das gegebenenfalls bis zu 3 Substituenten trägt, unabhängig ausgewählt aus Halogen, C₁₋₆-Alkyl, das 0-3 Fluorsubstituenten trägt, C₁₋₆-Alkoxy, das 0-3 Fluorsubstituenten trägt, und C₂₋₆-Alkenyl,
jedes R³ unabhängig Halogen, C₁₋₆-Alkyl, das 0-3 Fluorsubstituenten trägt, C₁₋₆-Alkoxy, das 0-3 Fluorsubstituenten trägt, oder C₂₋₆-Alkenyl bedeutet und
R⁴ und R⁵ unabhängig H oder Kohlenwasserstoff mit bis zu 12 Kohlenstoffatomen, das gegebenenfalls bis zu 3 Substituenten trägt, ausgewählt aus Halogen, Perfluor-C₁₋₄-alkyl, CN, Si(C₁₋₄-Alkyl)₃, OH, C₁₋₄-Alkoxy und OCF₃, bedeuten.

2. Eine Verbindung gemäß Anspruch 1, wobei Z CO₂H bedeutet.

3. Eine Verbindung gemäß Anspruch 1, wobei R² H oder Phenyl bedeutet, welches substituiert ist mit 2-CF₃, 3-CF₃, 4-CF₃, 2,4-Di(CF₃), 2-F-4-CF₃, 4-OCF₃, 4-Allyl, 4-n-Propyl, 4-Isopropyl oder 4-tert-Butyl.

4. Eine Verbindung gemäß Anspruch 3, wobei R² H oder 4-Trifluormethylphenyl bedeutet.

5. Eine Verbindung gemäß Anspruch 1, wobei (R³)ₙ 2-CF₃, 3-CF₃, 4-CF₃, 2,4-Di(CF₃), 2-F-4-CF₃, 4-OCF₃, 4-Allyl, 4-n-Propyl, 4-Isopropyl oder 4-tert-Butyl bedeutet.

6. Eine Verbindung gemäß Anspruch 1, wobei R⁴ und R⁵ unabhängig ausgewählt sind aus linearen oder verzweigten C₁₋₁₂-Alkyl-, Alkenyl- und Alkinylgruppen, C₃₋₆-alicyclischen Gruppen, C₃₋₆-alicyclischen C₁₋₆-Alkylgruppen, Phenylgruppen, Phenyl-C₁₋₆-alkylgruppen, Phenyl-C₂₋₄-alkinylgruppen und C₃₋₆-alicyclischen C₂₋₄-Alkinylgruppen und gegebenenfalls substituiert sind mit Halogen, C₁₋₄-Perfluoralkyl oder Si(C₁₋₄-Alkyl)₃.

7. Eine Verbindung gemäß Anspruch 1, die eine Verbindung der Formel II ist: 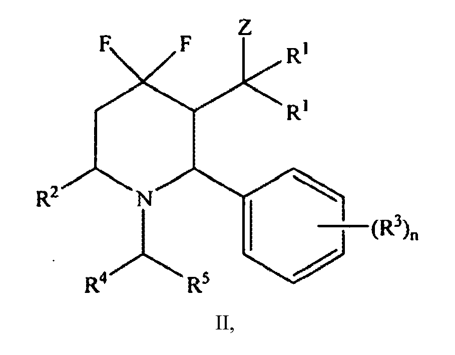 oder ein pharmazeutisch annehmbares Salz oder Hydrat davon,
wobei n, Z, R¹, R², R³, R⁴ und R⁵ wie in Anspruch 1 definiert sind.

8. Eine Verbindung gemäß Anspruch 7, wobei die relativen stereochemischen Konfigurationen der Substituenten am Piperidinring wie in Formel IIA gezeigt sind:

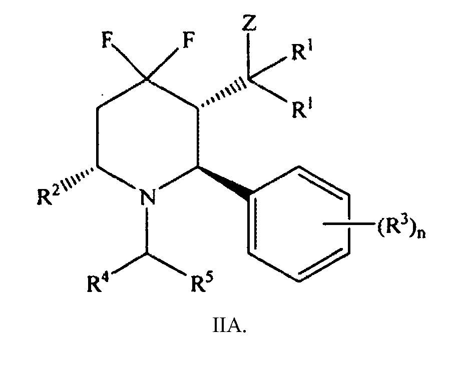

9. Eine Verbindung gemäß Anspruch 1, die eine Verbindung der Formel III ist: 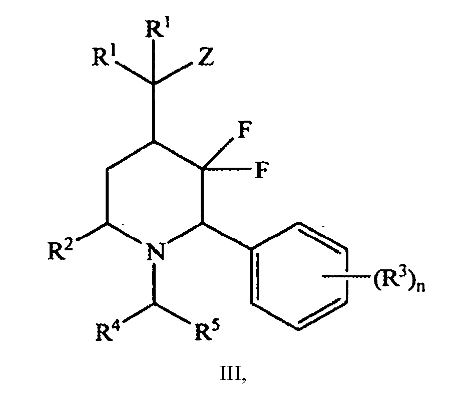 oder ein pharmazeutisch annehmbares Salz oder Hydrat davon,
wobei n, Z, R¹, R², R³, R⁴ und R⁵ wie in Anspruch 1 definiert sind.

10. Eine Verbindung gemäß Anspruch 9, wobei die relativen stereochemischen Konfigurationen der Substituenten am Piperidinring wie in Formel IIIA gezeigt sind:

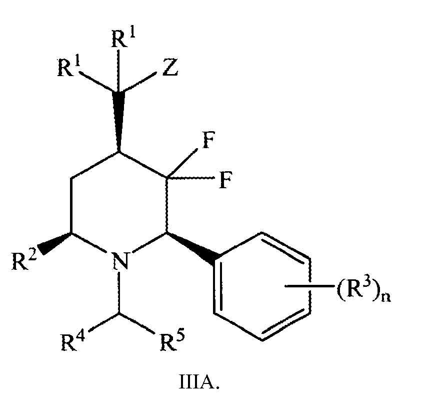

11. Eine pharmazeutische Zusammensetzung, die eine Verbindung gemäß einem vorhergehenden Anspruch und einen pharmazeutisch annehmbaren Träger enthält.

12. Eine Verbindung gemäß einem der Ansprüche 1-10 zur Verwendung bei der therapeutischen Behandlung des menschlichen Körpers.

13. Eine Verbindung gemäß Anspruch 12 zur Verwendung bei der Behandlung oder Prävention einer Erkrankung, die mit β-Amyloid-Ablagerung im Gehirn assoziiert ist.

14. Eine Verbindung gemäß Anspruch 13 zur Verwendung bei der Behandlung oder Prävention von Alzheimer-Krankheit.

## Revendications

1. Composé de la formule I: 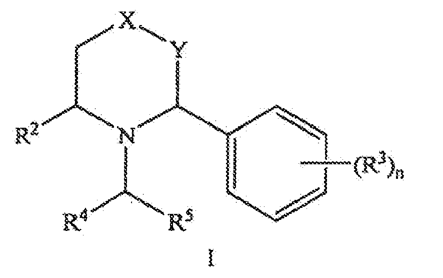 ou un sel ou hydrate pharmaceutiquement acceptable de celui-ci, où:
n est 0, 1, 2 ou 3;
l'un d'entre X et Y représente CF₂ et l'autre représente CH-C(R¹)₂-Z;
Z représente CO₂H ou un cycle tétrazole;
chaque R¹ représente indépendamment H ou un groupe hydrocarbure non aromatique de jusqu'à 6 atomes de carbone; ou bien les deux groupes R¹ complètent un groupe alicyclique C₃₋₆,
R² représente H ou phényle qui porte optionnellement jusqu'à 3 substituants sélectionnés indépendamment parmi halogène, alkyle C₁₋₆ portant 0-3 substituants fluor, alcoxy C₁₋₆ portant 0-3 substituants fluor et alcényle C₂₋₆;
chaque R³ représente indépendamment halogène, alkyle C₁₋₆ portant 0-3 substituants fluor, alcoxy C₁₋₆portant 0-3 substituants fluor ou alcényle C_{2-6;} et
R⁴ et R⁵ représentent indépendamment H ou un hydrocarbure de jusqu'à 12 atomes de carbone qui porte optionnellement jusqu'à 3 substituants sélectionnés parmi halogène, perfluoro-alkyle C₁₋₄, CN, Si(alkyle C₁₋₄)₃, OH, alcoxy C₁₋₄ et OCF₃.

2. Composé selon la revendication 1, dans lequel Z représente CO₂H.

3. Composé selon la revendication 1, dans lequel R² représente H ou phényle qui est substitué par 2-CF₃, 3-CF₃, 4-CF₃, 2,4-di(CF₃), 2-F-4-CF₃, 4-OCF₃, 4-allyle, 4-n-propyle, 4-isopropyle ou 4-tert-butyle.

4. Composé selon la revendication 3, dans lequel R² représente H ou 4-trifluorométhylphényle.

5. Composé selon la revendication 1, dans lequel (R³)ₙ représente 2-CF₃, 3-CF₃, 4-CF₃, 2,4-di(CF₃), 2-F-4-CF₃, 4-OCF₃, 4-allyle, 4-n-propyle, 4-isopropyle ou 4-tert-butyle.

6. Composé selon la revendication 1, dans lequel R⁴ et R⁵ sont sélectionnés indépendamment parmi des groupes alkyle C₁₋₁₂, alcényle et alcynyle linéaires ou ramifiés, des groupes alicyclique C₃₋₆, des groupes alicyclique C₃₋₆-alkyle C₁₋₆, des groupes phényles, des groupes phénylalkyle C₁₋₆, des groupes phénylalcynyle C₂₋₄ et des groupes alicyclique C₃₋₆-alcynyle C₂₋₄ et ils sont optionnellement substitués par halogène, perfluoroalkyle C₁₋₄ ou Si(alkyle C₁₋₄)₃.

7. Composé selon la revendication 1, qui est un composé de la formule II: 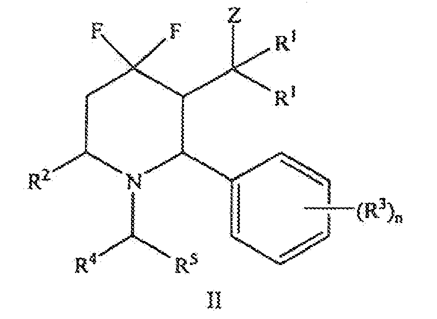 ou un sel ou hydrate pharmaceutiquement acceptable de celui-ci;
où n, Z, R¹, R², R³, R⁴ et R⁵ sont tels que définis dans la revendication 1.

8. Composé selon la revendication 7, dans lequel les configurations stéréochimiques relatives des substituants sur le cycle pipéridine sont comme il est illustré dans la formule IIA:

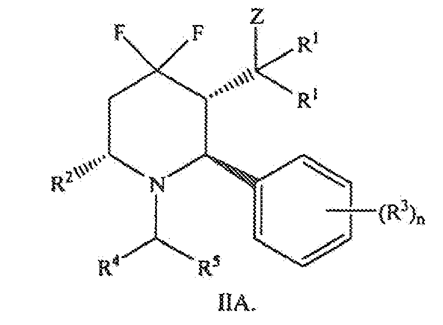

9. Composé selon la revendication 1 qui est un composé de la formule III: 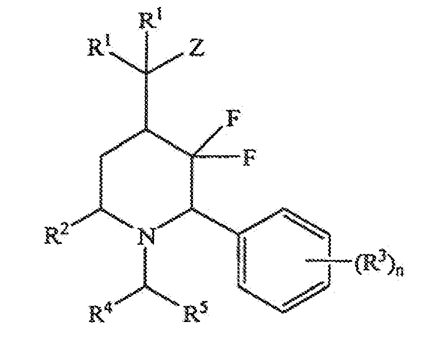 ou un sel ou hydrate pharmaceutiquement acceptable de celui-ci;
où n, Z, R¹, R², R³, R⁴ et R⁵ sont tels que définis dans la revendication 1.

10. Composé selon la revendication 9, dans lequel les configurations stéréochimiques relatives des substituants sur le cycle pipéridine sont comme il est illustré dans la formule IIIA:

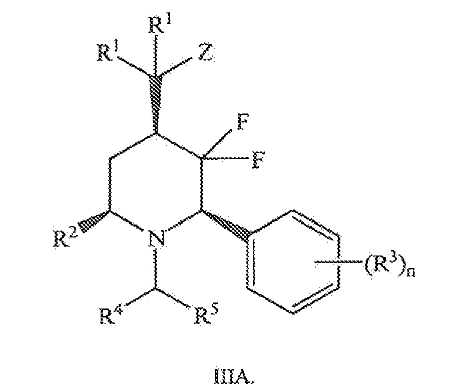

11. Composition pharmaceutique comprenant un composé selon l'une quelconque des revendications précédentes et un excipient pharmaceutiquement acceptable.

12. Composé selon l'une quelconque des revendications 1-10, à utiliser dans le traitement thérapeutique du corps humain.

13. Composé selon la revendication 12, qui est destiné à être utilisé dans le traitement ou la prévention d'une maladie associée au dépôt de β-amyloïde dans le cerveau.

14. Composé selon la revendication 13, qui est destiné à être utilisé dans le traitement ou la prévention de la maladie d'Alzheimer.
